# EUROPÄISCHE PATENTSCHRIFT

(11) **EP 4 394 426 B1**
(45) Veröffentlichungstag und Bekanntmachung des Hinweises auf die Patenterteilung: **29.01.2025**
(21) Anmeldenummer: 22217282.7
(22) Anmeldetag: 30.12.2022
(51) Int. Cl.: G01R 33/561, G01R 33/565, G01R 33/24, G01R 33/483

(54) **VERFAHREN UND VORRICHTUNG ZUR ANSTEUERUNG EINES MAGNETRESONANZBILDGEBUNGSSYSTEMS IM RAHMEN EINER ECHOPLANAR-BILDGEBUNG**
METHOD AND DEVICE FOR DRIVING A MAGNETIC RESONANCE IMAGING SYSTEM IN THE CONTEXT OF ECHO-PLANAR IMAGING
PROCÉDÉ ET APPAREIL DE COMMANDE D'UN SYSTÈME D'IMAGERIE PAR RÉSONANCE MAGNÉTIQUE DANS LE CADRE D'UNE IMAGERIE ECHOPLANAIRE

(43) Veröffentlichungstag der Anmeldung: 03.07.2024
(73) Patentinhaber: Siemens Healthineers AG, 91301 Forchheim (DE)
(72) Erfinder: Zeller, Mario, 91054 Erlangen (DE)
(74) Vertreter: Siemens Healthineers Patent Attorneys

(56) Entgegenhaltungen:
- CHI, D. ET AL.: "Field-Mapping-Embedded EPI for Geometric Distortion Correction", PROCEEDINGS OF THE JOINT ANNUAL MEETING ISMRM-ESMRMB 2022 & ISMRT ANNUAL MEETING, no. 1099, 22 April 2022 (2022-04-22), UK, XP040727647
- YARACH U. ET AL.: "Iterative SENSE with Integrated EPI Nyquist Ghost and Distortion Corrections", PROCECEEDINGS OF THE INTERNATIONAL SOCIETY FOR MAGNETIC RESONANCE IN MEDICINE, 25TH ANNUAL MEETING & EXHIBITION, no. 5157, 7 April 2017 (2017-04-07), USA, XP040692725
- SCOTT, H. ET AL.: "A Dual-Polarity Grappa Kernel for the Robust Reconstruction of Accelerated EPI data", 2015 IEEE 12TH INTERNATIONAL SYMPOSIUM ON BIOMEDICAL IMAGING (ISBI), IEEE, 16 April 2015 (2015-04-16), pages 1244 - 1247, XP033179632, DOI: 10.1109/ISBI.2015.7164099

## Beschreibung

Unabhängig vom grammatikalischen Geschlecht eines bestimmten Begriffes sind Personen mit männlicher, weiblicher oder anderer Geschlechteridentität mit umfasst.

Die Erfindung betrifft ein Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung gemäß einem Pulssequenzschema erfasst werden. Die Erfindung betrifft auch eine Steuereinrichtung für ein Magnetresonanzbildgebungssystem. Weiterhin betrifft die Erfindung ein Magnetresonanzbildgebungssystem.

Bildgebende Systeme, die auf einem Verfahren der Magnetresonanzmessung, insbesondere von Kernspins, basieren, sogenannte Magnetresonanztomographen, haben sich durch vielfältige Anwendungen erfolgreich etabliert und bewährt. Bei dieser Art der Bildakquisition wird meist ein statisches Grundmagnetfeld B₀, das zur Anfangsausrichtung und Homogenisierung von zu untersuchenden magnetischen Dipolen dient, zur Ortsauflösung des bildgebenden Signals mit einem schnell geschalteten Magnetfeld, dem sogenannten Gradientenfeld, überlagert. Zur Bestimmung von Materialeigenschaften eines abzubildenden Untersuchungsobjekts wird die Dephasierung bzw. Relaxationszeit nach einer Auslenkung der Magnetisierung aus der Anfangsausrichtung ermittelt, sodass verschiedene materialtypische Relaxationsmechanismen bzw. Relaxationszeiten identifiziert werden können. Die Auslenkung erfolgt meist durch eine Anzahl von HF-Pulsen (die Abkürzung HF steht für Hochfrequenz), auch als Anregungspulse bezeichnet, und die Ortsauflösung beruht dabei auf einer zeitlich festgelegten Manipulation der ausgelenkten Magnetisierung mit Hilfe des Gradientenfelds in einer sogenannten Messsequenz bzw. Ansteuersequenz, welche eine genaue zeitliche Abfolge von HF-Pulsen, der Änderung des Gradientenfeldes (durch Aussenden einer Schaltsequenz von Gradientenpulsen) sowie der Erfassung von Messwerten festlegt.

Typischerweise erfolgt eine Zuordnung zwischen gemessener Magnetisierung - aus der die erwähnten Materialeigenschaften abgeleitet werden können - und einer Ortskoordinate der gemessenen Magnetisierung im Ortsraum, in dem das Untersuchungsobjekt angeordnet ist, mit Hilfe eines Zwischenschritts. In diesem Zwischenschritt werden erfasste Magnetresonanz-Rohdaten, auch als k-Raumdaten bezeichnet, an Auslesepunkten im sogenannten "k-Raum" angeordnet, wobei die Koordinaten des k-Raums als Funktion des Gradientenfeldes kodiert sind. Der Betrag der Magnetisierung (insbesondere der Quermagnetisierung in einer Ebene quer zum vorbeschriebenen Grundmagnetfeld) an einem bestimmten Ort des Untersuchungsobjekts kann aus den Daten des Auslesepunkts mit Hilfe einer Fourier-Transformation ermittelt werden, die aus einer Signalstärke (Betrag der Magnetisierung), die einer bestimmten Frequenz (der Ortsfrequenz) bzw. Phasenlage zugeordnet ist, eine Signalstärke des Signals im Ortsraum berechnet.

Die Magnetresonanztomographie ist eine relativ langsam arbeitende Art eines bildgebenden Verfahrens, da die Daten entlang von Zeilen im Fourierraum bzw. im k-Raum sequentiell aufgenommen werden und für die Spin-Relaxation der angeregten Spins eine gewisse Zeit nicht unterschritten werden kann. Das Verfahren der Aufnahme von Bildern in zweidimensionalen Schichten ist im Vergleich zur Aufnahme in drei Dimensionen deutlich weniger fehleranfällig, weil die Zahl der Kodierungsschritte kleiner ist als bei einem dreidimensionalen Verfahren. Daher werden bei vielen Anwendungen Bildvolumen mit Stapeln von zweidimensionalen Schichten statt einer einzigen dreidimensionalen Aufnahme verwendet. Allerdings sind die Bildaufnahmezeiten aufgrund der langen Relaxationszeiten der Spins sehr lang, was beispielsweise für zu untersuchende Patienten eine Verringerung des Komforts bedeutet. Auch können die Patienten während der Aufnahme nicht kurz den Magnetresonanztomographen verlassen oder auch nur ihre Position ändern, da dies aufgrund der Positionsveränderung den Bildaufnahmevorgang zunichtemachen würde und der gesamte Prozess von vorne beginnen müsste. Folglich besteht ein wichtiges Ziel darin, die Aufnahme von zweidimensionalen Schichtstapeln zu beschleunigen.

Zur Beschleunigung der Bildaufnahme finden beispielsweise parallele Bildaufnahmetechniken Anwendung. Bei einigen der Bildaufnahmetechniken können Artefakte aufgrund von Unterabtastungen auftreten. Diese Artefakte können durch Anwendung von Rekonstruktionsalgorithmen eliminiert werden.

Zur beschleunigten Aufnahme von Magnetresonanzrohdaten wird häufig die Echoplanar-Bildgebung (abgekürzt mit EPI bzw. EPI-Pulssequenz) genutzt. Dabei werden pro Repetition eine Vielzahl von Echos, auch als Echo-Train oder Echozug bezeichnet, durch Hin- und Herschalten von Frequenzgradienten, auch als Auslesegradienten bezeichnet, erfasst. Somit kann ein Bild mit einer geringen Anzahl von Anregungen bzw. Repetitionen aufgezeichnet werden.

Echoplanar-Bildgebungssequenzen werden oft in Form von Mehrschichtbildgebungssequenzen angewendet, um die Bildgebung zu beschleunigen und beispielsweise auch Aufnahmen von bewegten Objekten zu ermöglichen. Echoplanar-Bildgebungssequenzen umfassen trotz des beschriebenen Echozugs typischerweise eine Vielzahl von Repetitionen. Unter einer Repetition versteht man einen Pulssequenzabschnitt zwischen zwei Anregungs-HF-Pulsen. Besonders bei einer sogenannten BOLD-Bildgebung (BOLD = blood oxygenation level dependent = "abhängig vom Blutsauerstoffgehalt", diese Art der Bildgebung veranschaulicht den Sauerstoffgehalt in den roten Blutkörperchen, womit zum Beispiel die Hirnaktivität eines Patienten dargestellt werden kann) und erweiterten Diffusionsprotokollen, wie MDDW (MDDW = multi-directional diffusion weighting = diffusionsgewichtete Bildgebung in mehreren Richtungen), sind Aufnahmen mit mindestens 60 Repetitionen keine Seltenheit.

Üblicherweise werden bei den einzelnen Repetitionen direkt nach dem Anregungspuls im Rahmen einer sogenannten Navigator-Aufnahme jeweils drei Linien zur Korrektur von Nyquist-Ghosting und/oder zur Korrektur einer B₀-Drift aufgenommen. Auf diese Weise ergibt sich bei der erwähnt hohen Anzahl von Repetitionen eine sehr hohe zeitliche Auflösung für die Korrekturen der genannten Artefakte.

Wie bereits erwähnt, werden EPI-Aufnahmen mit parallelen Bildgebungstechniken, wie zum Beispiel GRAPPA (GRAPPA = Gene-Ralized Autocalibrating Partially Parallel Acquisitions = verallgemeinerte selbstkalibrierende teilweise parallele Akquisitionen) oder SMS (SMS = Simultaneous Multi-Slice = Simultane Mehrschichtbildgebung), durchgeführt, um mehrere Schichten simultan bildlich zu erfassen.

Daneben gibt es erweiterte Korrekturmethoden, wie zum Beispiel "Dual Polarity" (siehe Hoge et al., "Dual-Polarity GRAPPA for Simultaneous Reconstruction and Ghost Correction of Echo Planar Imaging Data", Magnetic Resonance in Medicine 2016 Jul; 76(1):32-44. Doi: 10.1002/mrm.25839. Epub 2015 Jul 24) zur besonders genauen Korrektur von Geisterartefakten oder räumlich aufgelöste Eddy Current-Korrekturverfahren (siehe Chen et al., "Single-Shot and Segmented EPI Ghost Artifacts Removal with Two-Dimensional Phase Correction", Proc. Intl. Soc. Magn. Reson. Med. 8 (2000)). Bei diesen genannten speziellen Verfahren werden Navigatordaten für die erwähnten Korrekturverfahren und zusätzlich Referenzdaten für eine Ergänzung von unterabgetasteten k-Raumdaten benötigt. Damit erhöht sich die Anzahl von erforderlichen Repetitionen der Pulssequenz und somit auch die Messzeit bzw. die Zeit für ein solches EPI-Bildaufnahmeverfahren.

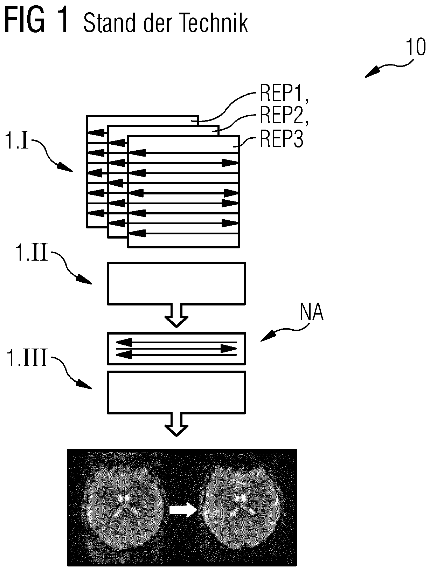

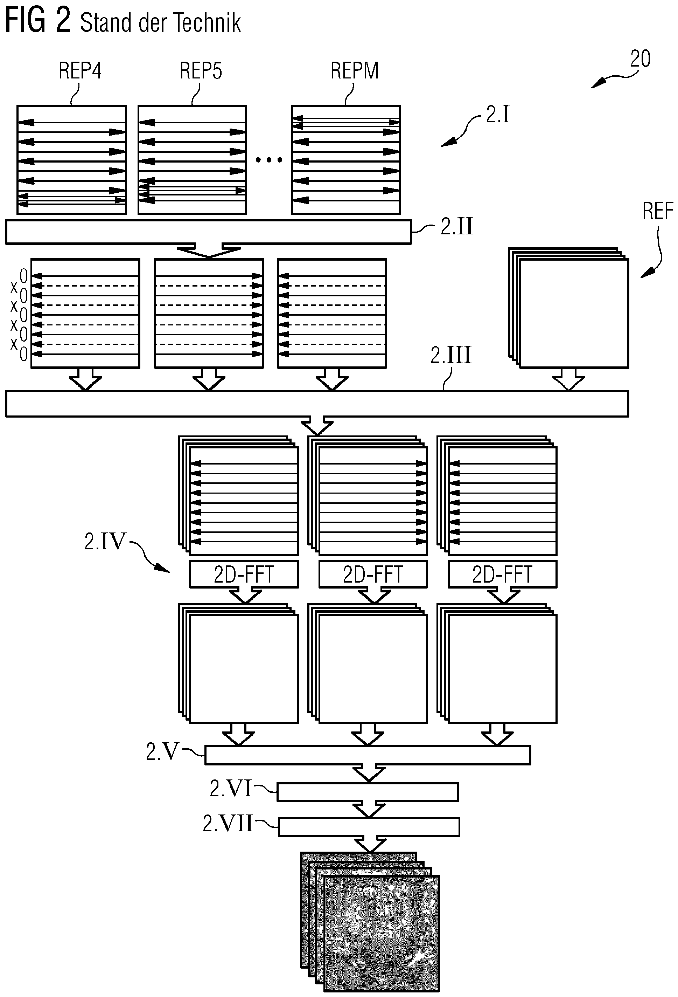

In Chi et al., "Field-Mapping-Embedded EPI for Geometric Distortion Correction", Proc. Intl. Soc. Mag. Reson. Med. 30 (2022) 1099, wird ein Verfahren beschrieben, bei dem die vorhandenen drei Linien der Navigatoraufnahme zur Erfassung von Nyquist-Ghosting-Korrekturdaten so modifiziert werden und über die Dauer der gesamten Aufnahme fortgesetzt werden, dass in jeder Repetition ein anderer aufzunehmender k-Raum-Abschnitt kodiert wird. Die erfassten k-Raum-Abschnitte werden zu passenden k-Räumen zusammengesetzt und es lassen sich über die Dauer der Aufnahme daraus B₀-Feldkarten zur Korrektur geometrischer Verzerrungen gewinnen. Die Polarität der Auslesegradienten zur Erfassung der Korrekturdaten wird in kx-Richtung (Ausleserichtung) gespiegelt, so dass k-Raumdaten in drei unipolaren k-Räumen für die spätere bildbasierte Berechnung von B₀-Feldkarten gewonnen werden. Zur Erzeugung der B₀-Feldkarten werden zusätzlich Referenzscans zur Anwendung paralleler Bildgebungsverfahren benötigt. Das Verfahren ist in FIG 1 und FIG 2 dargestellt.

In US 10 162 037 B2 und US 11 280 870 B2 werden Verfahren beschrieben, bei denen ein Aufnahmeschema der drei Linien der Navigator-Aufnahme derart modifiziert wird, dass für eine SMS-Bildgebung schichtspezifische Ghost-Korrekturdaten erhalten werden.

Es ist somit eine Aufgabe der vorliegenden Erfindung, ein zeitsparendes beschleunigtes Echoplanar-Bildgebungsverfahren mit einer Korrektur der sogenannten Nyquist-Ghost-Effekte und mit im Vergleich zu herkömmlichen Verfahren reduzierter Aufnahmezeit anzugeben.

Diese Aufgabe wird durch ein Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung gemäß einem Pulssequenzschema erfasst werden, gemäß Patentanspruch 1, durch eine Steuereinrichtung für ein Magnetresonanzbildgebungssystem gemäß Patentanspruch 12 und durch ein Magnetresonanzbildgebungssystem gemäß Patentanspruch 13 gelöst.

Das erfindungsgemäße Verfahren weist das Abtasten des k-Raums gemäß einem Pulssequenzschema auf. Dieses Pulssequenzschema weist eine Mehrzahl von Repetitionen auf, welche jeweils ein erstes Abtastschema für eine Aufnahme von k-Raumdaten zur Nyquist-Ghosting-Korrektur und/oder zur Erzeugung von Magnetfeldkarten zur Magnetfeldkorrektur, vorzugsweise zur B₀-Magnetfeldkorrektur bzw. zur Erzeugung sogenannter B₀-Feldkarten, ein nachfolgendes zweites Abtastschema für eine beschleunigte Echoplanar-Aufnahme und ein für beide Aufnahmen gemeinsames Anregungsschema umfassen. Magnetfeldkarten geben die Abweichung der Feldstärke von bei der Magnetresonanzbildgebung erzeugten Magnetfeldern von Nennwerten an. Diese Abweichungen führen zu Phasenabweichungen der Messsignale, welche bei der Bildrekonstruktion berücksichtigt werden müssen. Als Abtastschema soll die spezifische Art und Weise verstanden werden, mit der der k-Raum mit Hilfe von Gradientenschaltungen abgetastet wird, wobei als Echosignale bzw. Messsignale Hochfrequenzsignale empfangen werden. Insbesondere korrespondiert mit einem solchen Abtastschema eine spezifische k-Raum-Trajektorie und eine spezifische Abfolge von Gradientenpulsen bzw. eine spezifische Gradientenpulssequenz oder Gradientenpulsteilsequenz. Als k-Raumdaten sind die Messsignale zu verstehen, die spezifischen Punkten im k-Raum zugeordnet sind. Das unmodifizierte erste Abtastschema ist unkodiert, d.h., das Abtasten erfolgt ohne Phasenkodierung und vorzugsweise wird somit die "nullte" k-Raumzeile mehrmals, besonders bevorzugt dreimal in wechselnder Richtung abgetastet bzw. erfasst. Als beschleunigte Echoplanar-Aufnahme soll eine Echoplanar-Aufnahme verstanden werden, bei der durch eine Unterabtastung Zeit bei der Aufnahme von k-Raumdaten eingespart wird.

Das erste Abtastschema ist in einer echten Teilmenge der Mehrzahl von Repetitionen dahingehend modifiziert, dass auf Basis der mit dem modifizierten ersten Abtastschema erfassten k-Raumdaten eine Ergänzung von in Folge der Unterabtastung fehlender Signalinformation ermöglicht wird. Insbesondere soll durch die mit dem modifizierten ersten Abtastschema erfassten k-Raumdaten eine Ergänzung von in Folge der Unterabtastung fehlender k-Raumdaten ermöglicht werden und/oder eine Korrektur im Bildraum von aufgrund der Unterabtastung auftretender Artefakten ermöglicht werden.

Als echte Teilmenge ist eine Teilmenge der Mehrzahl von Repetitionen zu verstehen, die mindestens eine der Repetitionen nicht aufweist. Dadurch, dass die Modifikation nicht in allen Repetitionen erfolgt, gibt es auch eine Anzahl, genauer gesagt mindestens eine Repetition der Mehrzahl von Repetitionen, bei der bzw. bei denen die Modifikation nicht auftritt und die daher zur Akquisition der k-Raumdaten zur Nyquist-Ghosting-Korrektur genutzt wird bzw. werden. Das modifizierte Abtastschema ist derart ausgestaltet, dass damit zusätzliche k-Raumdaten erfasst werden, die eine Basis für eine Ergänzung der durch die beschleunigte Bildgebung fehlenden Signalinformation bilden. Wie bereits erwähnt, kann diese fehlende Signalinformation insbesondere fehlende k-Raumdaten in unterabgetasteten k-Raumbereichen umfassen. Die fehlende Signalinformation kann aber auch die aufgrund der fehlenden k-Raumdaten verursachten Artefakte im Bildraum umfassen. Für die Akquisition eines Teils des k-Raums ist daher das modifizierte erste Abtastschema im Gegensatz zu dem unmodifizierten ersten Abtastschema vorzugsweise kodiert, d.h. es werden Phasenkodiergradienten geschaltet, um Phasenkodierlinien mit unterschiedlicher Phase zu akquirieren.

Wie später noch im Einzelnen erläutert, werden zur Berechnung der fehlenden k-Raumdaten auf Basis der zusätzlichen k-Raumdaten Informationen über das Messverhalten des Magnetresonanzsystems gewonnen, mit denen die fehlenden k-Raumdaten rekonstruiert werden können. Insbesondere können bei sogenannten Mehrspulensystemen, mit denen beschleunigte Bildgebungsverfahren üblicherweise durchgeführt werden, durch eine sogenannte Autokalibration Informationen darüber gewonnen werden, mit welcher Gewichtung bzw. welchen Gewichten Signale von unterschiedlichen Spulen von einem Punkt im k-Raum addiert werden müssen, um Signale benachbarter, möglicherweise nicht abgetasteter Punkte im k-Raum zu berechnen.

Auf Basis dieser Gewichte und der unterabgetasteten k-Raumdaten können also Schlüsse auf die Signalintensität in den nicht abgetasteten Teilbereichen des k-Raums gezogen werden. Hierzu werden auf Basis der bei der Autokalibration ermittelten Information vorzugsweise sogenannte Kernel berechnet, welche die erwähnten Gewichtungen umfassen. Werden diese Kernel auf die unterabgetasteten k-Raumdaten angewendet, so lassen sich die aufgrund der Unterabtastung fehlenden Signalinformationen ermitteln.

Alternativ kann die Ergänzung der fehlenden Signalinformation auch im Bildraum erfolgen, wobei Spulensensitivitätswerte ermittelt werden, die eine Berechnung artefaktfreier Bilddaten auf Basis von mit unterschiedlichen Spulen durch Unterabtastung gewonnenen artefaktbehafteten Bilddaten ermöglichen.

Bei der Rekonstruktion der fehlenden Signalinformation im Bildraum, wie es zum Beispiel bei dem SENSE-Verfahren erfolgt, werden die mit dem modifizierten ersten Abtastschema gewonnen k-Raumdaten zur Ermittlung von Spulensensitivitätswerten genutzt. Außerdem werden zunächst für unterschiedliche Spulen auf Basis der unterabgetasteten k-Raumdaten jeweils artefaktbehaftete Bilddaten mit einem eingeschränkten Bildfeld (FOV = Field of View) rekonstruiert. Bei diesen Bilddaten tritt eine typische Einfaltung überstehender Bildanteile auf. Auf Basis der Spulensensitivitätswerte und der artefaktbehafteten Bilddaten mit eingeschränktem Bildfeld lassen sich schließlich artefaktfreie Bilddaten des vollständigen Bildfelds (FOV) berechnen.

Während also die Navigatordaten bzw. k-Raumdaten für die Nyquist-Ghosting-Korrektur oder B₀-Magnetfeldkorrektur unkodiert bzw. nicht phasenkodiert erfasst werden, erfolgt bei der Aufnahme von k-Raumdaten gemäß dem modifizierten ersten Abtastschema eine Kodierung, vorzugsweise Phasenkodierung. Diese Phasenkodierung erlaubt die erwähnte Autokalibration, welche insbesondere eine Abtastung eines Teilbereichs des k-Raums mit im Vergleich zu der Unterabtastung erhöhten Auflösung, vorzugsweise mit einer vollständigen Abtastung, umfasst. Auf Basis dieser höher aufgelöst abgetasteten k-Raumdaten lassen sich fehlende k-Raumdaten bzw. lässt sich fehlende Signalinformation ermitteln, wobei die Rekonstruktion der fehlenden Signalinformation sowohl im k-Raum, wie zum Beispiel beim GRAPPA-Verfahren, als auch durch eine Berechnung im Bildraum, wie zum Beispiel beim SENSE-Verfahren, erfolgen kann. Bildraumdaten und k-Raumdaten können durch eine Fouriertransformation ineinander überführt werden.

Bei dem gemeinsamen Anregungsvorgang wird vorzugsweise ein erster Schichtselektionsgradientenpuls in Schichtselektionsrichtung erzeugt. Zudem wird ein Anregungs-HF-Puls erzeugt, welcher zur Anregung einer oder mehrerer, vorzugsweise N simultan anzuregender Schichten entsprechend N Anregungsfrequenzen umfasst, wobei N eine ganze natürliche Zahl ist. Wie später noch ausführlicher erläutert, weist N meist den Wert 2 oder maximal 8 auf.

Wie bereits erwähnt, umfasst der Auslesevorgang zum Erfassen der k-Raumdaten in der Regel das alternierende Schalten von Rephasierer-Gradientenpulsen in Ausleserichtung, wobei zusätzlich weitere Gradientenpulse, beispielsweise in Phasenrichtung und/oder in Schichtselektionsrichtung, erzeugt werden und HF-Signale zur Akquisition von Magnetresonanzrohdaten bzw. der k-Raumdaten empfangen werden.

Das erfindungsgemäße Verfahren nutzt die Erkenntnis, dass die zeitliche Auflösung für die Erfassung von sogenannten Navigatordaten für die Nyquist-Ghosting-Korrektur oder die B₀-Feld-korektur nicht allzu hoch sein muss, da die zeitliche Entwicklung von Störeinflüssen in der Regel langsam stattfindet. Daher können einige der Pulssequenzabschnitte, die herkömmlich für die Ermittlung von Korrekturdaten für das Nyquist-Ghosting oder die B₀-Feldkorrektur genutzt werden, für die Akquisition von k-Raumdaten als Referenzdaten quasi umfunktioniert werden. Diese Referenzdaten werden für die Korrektur von aufgrund der Unterabtastung auftretenden Artefakten genutzt.

Erfolgt eine Kompensation der Unterabtastung im k-Raum, wie zum Beispiel beim GRAPPA-Verfahren, so werden die Referenzdaten für die Berechnung von fehlenden k-Raumdaten für die eigentliche Bildgebung genutzt. Da die Beschleunigung einer Echoplanar-Aufnahme auf einer Unterabtastung des k-Raums basiert, müssen die aufgrund der Unterabtastung für eine vollständige Bildinformation fehlenden k-Raumdaten auf Basis von Referenzdaten geschätzt bzw. näherungsweise berechnet werden.

Vorteilhaft muss nun anders als bei einer herkömmlichen Vorgehensweise kein separater Aufnahmevorgang für die Erfassung der Referenzdaten erfolgen, wodurch die Gesamtzeit für eine Bildgebung reduziert wird. Außerdem braucht die Zeitdauer einer Repetition oder eines Echozugs nicht erhöht zu werden, da die Aufnahme der Referenzdaten in einigen Repetitionen einfach die Aufnahme der Navigatordaten ersetzen und so die Echozeit und die Repetitionszeit der einzelnen Repetitionen vorzugsweise gleich bleibt bzw. unverändert bleiben kann, unabhängig davon, ob mit dem ersten Abtastschema Navigatordaten bzw. Daten für eine Korrektur der Nyquist-Ghost-Effekt oder Referenzdaten erfasst werden.

Im Vergleich zu einer herkömmlichen EPI-Pulssequenz, bei der eine separate Aufnahme von Referenzdaten erfolgt, wird die Bildaufnahmedauer bzw. die Gesamtzeit zur Akquisition von k-Raumdaten also vorteilhaft reduziert.

Zudem betrifft die Erfindung eine Ansteuersequenz zur Ansteuerung eines Magnetresonanzbildgebungssystems. Die Ansteuersequenz weist Ansteuersignale entsprechend einem Pulssequenzschema mit einer Mehrzahl von Pulssequenzabschnitten bzw. Repetitionen mit jeweils einem Anregungsabschnitt und einem Ausleseabschnitt auf.

Die Repetitionen umfassen als ersten Pulssequenzteilabschnitt das erwähnte gemeinsame Anregungsschema, als zweiten Pulssequenzteilabschnitt das ebenfalls bereits erwähnte erste Abtastschema für eine Aufnahme von k-Raumdaten zur Nyquist-Ghosting-Korrektur oder zur Erzeugung von Magnetfeldkarten zur Magnetfeldkorrektur, insbesondere zur B₀-Feldkorrektur, und als dritten Pulssequenzteilabschnitt das bereits erwähnte nachfolgende zweite Abtastschema für eine beschleunigte Echoplanar-Aufnahme. Das erste Abtastschema einer echten Teilmenge der Mehrzahl von Repetitionen ist dahingehend modifiziert, dass auf Basis der mit dem modifizierten Abtastschema erfassten k-Raumdaten eine Ergänzung von in Folge der Unterabtastung fehlenden k-Raumdaten und/oder eine Korrektur im Bildraum von aufgrund der Unterabtastung auftretenden Artefakten ermöglicht wird. Die erfindungsgemäße Ansteuersequenz teilt die Vorteile des erfindungsgemäßen Verfahrens zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden.

Die erfindungsgemäße Steuereinrichtung für ein Magnetresonanzbildgebungssystem ist zur Steuerung des Magnetresonanzbildgebungssystems unter Nutzung des erfindungsgemäßen Verfahrens zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, ausgebildet. Die erfindungsgemäße Steuereinrichtung teilt die Vorteile des erfindungsgemäßen Verfahrens zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung gemäß einem Pulssequenzschema erfasst werden.

Das erfindungsgemäße Magnetresonanzbildgebungssystem weist die erfindungsgemäße Steuereinrichtung auf. Das erfindungsgemäße Magnetresonanzbildgebungssystem teilt die Vorteile des erfindungsgemäßen Verfahrens zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung gemäß einem Pulssequenzschema erfasst werden.

Ein Großteil der zuvor genannten Komponenten des erfindungsgemäßen Verfahrens zur Ansteuerung eines Magnetresonanzbildgebungssystems kann ganz oder teilweise in Form von Softwaremodulen in einem Prozessor eines entsprechenden Rechensystems realisiert werden, z. B. von einer Steuereinrichtung eines Magnetresonanztomographiesystems oder einem Computer, der zur Steuerung eines solchen Systems genutzt wird. Eine weitgehend softwaremäßige Realisierung hat den Vorteil, dass auch schon bisher verwendete Rechensysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem ladbar ist, mit Programmabschnitten, um die Schritte des erfindungsgemäßen Verfahrens zur Ansteuerung eines Magnetresonanzbildgebungssystems auszuführen, wenn das Programm in dem Rechensystem ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zum Rechensystem bzw. zur Steuereinrichtung und/oder zur Speicherung an oder in dem Rechensystem bzw. der Steuereinrichtung kann ein computerlesbares Medium, z. B. ein Memorystick, eine Festplatte oder ein sonstiger transportabler oder fest eingebauter Datenträger dienen, auf welchem die von einem Rechensystem einlesbaren und ausführbaren Programmabschnitte des Computerprogramms gespeichert sind. Das Rechensystem kann z. B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

In einer bevorzugten Ausgestaltung des erfindungsgemäßen Verfahrens zur Ansteuerung eines Magnetresonanzbildgebungssystems bleibt bei der Modifikation des ersten Abtastschemas die Zeitdauer der von der Modifikation betroffenen Repetition und vorzugsweise auch die Zeitdauer des zweiten Pulssequenzteilabschnitts, welcher das erste Abtastschema wiedergibt, unverändert. Vorteilhaft wird eine Erhaltung eines stationären Zustands der hinsichtlich ihrer Magnetisierung ausgelenkten Spins, auch als "Steady State" bezeichnet, aufrechterhalten. Ein solcher "Steady State" wird insbesondere bei Pulssequenzen benötigt, bei denen die Repetitionszeit oder die Echozeit kürzer ist als die Relaxationszeit der angeregten Spins. Vorteilhaft werden Bildartefakte, welche durch eine Beeinträchtigung des stationären Zustands verursacht werden würden, vermieden.

Wird in einer Variante des erfindungsgemäßen Verfahrens zur Ansteuerung eines Magnetresonanzbildgebungssystems durch die Modifikation des ersten Abtastschemas eine Änderung der Zeitdauer der betroffenen Repetitionen bewirkt, so kann es erforderlich bzw. vorteilhaft sein, diese Änderung der Zeitdauer durch sogenannte Dummy-Pulse bzw. Präparationspulse auszugleichen, um eine korrekte Erfassung von k-Raumdaten bzw. HF-Resonanzsignalen zu ermöglichen.

Beispielsweise wird bei einer solchen Modifikation des ersten Abtastschemas mit geänderter Zeitdauer die Anzahl der pro Repetition abgetasteten k-Raumlinien bei dem modifizierten ersten Abtastschema im Vergleich zu dem unmodifizierten ersten Abtastschema erhöht, um auf diese Weise einen vergrößerten k-Raumbereich für die Gewinnung der Referenzdaten abtasten zu können.

Besonders bevorzugt umfassen alle Pulssequenzabschnitte bzw. Repetitionen der Mehrzahl von Repetitionen der zur Bildgebung genutzten EPI-Pulssequenz entweder das erste Abtastschema oder das modifizierte erste Abtastschema. Vorteilhaft wird eine maximale Menge an k-Raumdaten für die Korrektur der Artefakte und die Ergänzung der unterabgetasteten k-Raumdaten akquiriert.

Ganz besonders bevorzugt ist es, wenn nur genau eine einzige dieser Repetitionen, vorzugsweise die erste Repetition, das erste Abtastschema aufweist und die übrigen bzw. alle übrigen Repetitionen das modifizierte erste Abtastschema aufweisen, um die Menge der k-Raumdaten, welche für die Ergänzung der unterabgetasteten k-Raumdaten verwendet werden, zu maximieren. Bei dieser Variante wird die resultierende Bildqualität aufgrund der breiteren Datenbasis für die Ermittlung der aufgrund der Unterabtastung fehlenden k-Raumdaten weiter verbessert. Bei dieser Variante wird nur die erste Repetition für eine Aufnahme von unkodierten k-Raumdaten, vorzugsweise Navigatordaten bzw. entsprechende k-Raumdaten für eine Korrektur von Nyquist-Ghost-Korrekturen und/oder B₀-Feld-Korrekturen genutzt.

Es kann aber auch sehr vorteilhaft sein, mehrere über das gesamte Pulssequenzschema verteilte Repetitionen für eine Aufnahme von unkodierten k-Raumdaten, vorzugsweise Navigatordaten für eine Korrektur von Nyquist-Ghost-Korrekturen und/oder B₀-Feldkorrekturen zu nutzen, um Korrekturfaktoren für eine solche Korrektur zu aktualisieren. Vorteilhaft können die Korrekturfaktoren an dynamische Veränderungen, welche das Auftreten der Artefakte, wie zum Beispiel Nyquist-Ghosting-Strukturen, beeinflussen, angepasst werden.

Bevorzugt wird das erste Abtastschema in einer EPI-Pulssequenz in unterschiedlichen Repetitionen durch unterschiedlich modifizierte Abtastschemata ersetzt.

Hierbei werden in unterschiedlichen Repetitionen unterschiedlich modifizierte erste Abtastschemata zur Abtastung des **k-**Raums genutzt. Beispielsweise müssen einzelne Gradienten zur Abtastung unterschiedlicher k-Raumbereiche variieren. Außerdem kann es auch nötig sein, wie später noch im Einzelnen erläutert wird, dass eine Mehrzahl von unterschiedlich strukturierten, mit unterschiedlichen Abtastschemata aufgenommenen Kerneln für die Berechnung der fehlenden k-Raumdaten benötigt wird, wenn zum Beispiel unterschiedliche Aufnahmetechniken kombiniert werden oder bestimmte Artefakte besonders exakt korrigiert werden sollen.

Bei einer bevorzugten Variante des erfindungsgemäßen Verfahrens zur Ansteuerung eines Magnetresonanzbildgebungssystems werden das modifizierte erste Abtastschema und die Anzahl der von der echten Teilmenge umfassten Repetitionen so gewählt, dass eine vollständige Abtastung eines k-Raum-Teilbereichs, der mindestens für eine vollständige Ergänzung der fehlenden k-Raumdaten benötigt wird, erfolgt. Für die Ermittlung der bei der Unterabtastung zunächst noch fehlenden k-Raumdaten werden bevorzugt sogenannte Kernel auf der Basis von **k-**Raumdaten berechnet, die aus einem k-Raumbereich stammen, der vollständig abgetastet wurde. In diesem Fall ist das der Bereich des k-Raums, der mit dem modifizierten ersten Abtastschema abgetastet wird.

Typische Werte für eine Erzeugung eines Kernels für eine Ergänzung von k-Raumdaten in einem unterabgetasteten Bildaufnahmeverfahren sind 24 Phasenraumkodierlinien, für die bei der Wahl eines GRAPPA-Abtastschemas 8 Repetitionen oder eines SMS-Abtastschemas 16 Repetitionen benötigt werden, wenn das erste Abtastschema für die Abtastung von 3 Phasenraumkodierlinien ausgelegt ist und bei der Modifikation an dieser Anzahl nichts geändert wird. Bei der Wahl einer Abtastung von M Phasenraumkodierlinien für die Korrektur der Nyquist-Ghost-Effekte (M ist eine natürliche Zahl) werden entsprechend 24/M modifizierte Repetitionen für das GRAPPA-Abtastschema benötigt und 48/M modifizierte Repetitionen für das SMS-Abtastschema benötigt.

Mithin umfasst bei dem erfindungsgemäßen Verfahren das modifizierte erste Abtastschema bevorzugt eine Abtastung von mindestens drei k-Raumlinien in Ausleserichtung mit unterschiedlicher Phasenkodierung. Vorteilhaft genügen drei k-Raumlinien bei der N/2-Nyquist-Ghost-Korrektur für eine Korrektur in drei Raumrichtungen. Bei der Modifikation des ersten Abtastschemas braucht bei dieser vorteilhaften Variante die Anzahl der drei Gradientenpulse in Ausleserichtung nicht geändert zu werden. Es soll an dieser Stelle erwähnt werden, dass das erste Abtastschema auch nur die Abtastung von ein oder zwei k-Raumlinien oder mehr als drei k-Raumlinien in Ausleserichtung umfassen kann. Eine Abtastung von k-Raumlinien gemäß dem ersten unmodifizierten Abtastschema soll insbesondere ausdrücklich die Variante umfassen, bei der die Abtastung derselben k-Raumlinie möglicherweise mehrmals in beiden Richtungen erfolgt und besonders bevorzugt unkodiert erfolgt. Soll sich die Anzahl der abgetasteten k-Raumlinien des modifizierten ersten Abtastschemas von der Anzahl der abgetasteten **k-**Raumlinien des unmodifizierten ersten Abtastschemas unterscheiden, so sind gegebenenfalls noch zusätzliche, bereits erwähnte Modifikationen als Ausgleich sinnvoll, um die Echozeit und die Repetitionszeit der modifizierten Repetitionen im Vergleich zu den unmodifizierten Repetitionen nicht oder nicht zu sehr zu ändern.

Besonders bevorzugt umfasst das modifizierte erste Abtastschema des erfindungsgemäßen Verfahrens eine Abtastung von vorzugsweise mindestens drei k-Raumlinien in Ausleserichtung mit zumindest teilweise unterschiedlicher Schichtkodierung.

Diese unterschiedliche Schichtkodierung ist insbesondere bei Bildaufnahmeverfahren mit simultaner Schichtbildgebungstechnik, insbesondere für SMS, GRAPPA + SMS und Dual Polarity GRAPPA, aus den vorstehend genannten Gründen sehr effektiv, um die Bildqualität zu verbessern.

Ganz besonders bevorzugt wird für das modifizierte erste Abtastschema und das zweite Abtastschema ein analog strukturiertes Abtastschema, anders ausgedrückt ein Abtastschema desselben Typs verwendet. Wird für die Erzeugung der Referenzdaten, also der k-Raumdaten, auf deren Basis unterabgetastete k-Raumdaten ergänzt werden sollen, und für die Abtastung der unterabgetasteten k-Raumdaten selbst ein Abtastschema desselben Typs verwendet, so können Störeffekte sowie geometrische Verzerrungen vermieden bzw. reduziert werden, die bei unterschiedlichen Abtastschemata für die Akquisition der Referenzdaten und der unterabgetasteten k-Raumdaten auftreten würden. Ein analoges Abtastschema ist hierbei dadurch definiert, dass für zwei zeitlich nacheinander abgetastete k-Raum-Linien der Abstand im k-Raum bei den aufgenommenen Referenzdaten in einer Repetition und unterabgetasteten Daten gleich ist oder anders ausgedrückt, dass die Referenzdaten in einer Repetition die gleiche Unterabtastung aufweisen wie die unterabgetasteten (Bildgebungs-)Daten. Die Referenzdaten aus allen Repetitionen zusammengesetzt ergeben dann aber natürlich wieder einen voll abgetasteten k-Raum.

In einer besonders effektiven Ausgestaltung des erfindungsgemäßen Verfahrens zur Ansteuerung eines Magnetresonanzbildgebungssystems erfolgt zu unterschiedlichen Zeitpunkten der beschleunigten Echoplanar-Bildgebung eine Aktualisierung der mit dem modifizierten ersten Abtastschema erfassten k-Raumdaten bzw. Referenzdaten. Vorteilhaft kann die Datenbasis für die Ergänzung der unterabgetasteten k-Raumdaten an dynamische Effekte, insbesondere Störeinflüsse, wie zum Beispiel Phasenänderungen oder Patientenbewegungen, angepasst werden. Diese Anpassung erlaubt vorteilhaft eine über den ganzen Verlauf des Bildgebungsvorgangs gleichbleibende Bildqualität.

Hierzu kann eine vollständige Abtastung der Referenzdaten bis zu einem ersten Zeitpunkt erfolgen und diese vollständige Abtastung zu einem späteren Zeitpunkt einmal oder mehrmals wiederholt werden. Bildlich gesprochen kann auf Basis der Mehrzahl von Referenzdatensätzen ein gleitender Durchschnitt von Referenzdaten gebildet werden, auf deren Basis die zu ergänzenden k-Raumdaten ermittelt werden. Alternativ kann auch eine Ermittlung von "stabilen" bzw. konstanten Referenzdaten nach mehrmaliger Aufnahme der Referenzdaten erfolgen, wenn eine Mittelung über mehrere Referenzdatensätze erst nach Aufnahme sämtlicher Referenzdatensätze erfolgt bzw. die Rekonstruktion der fehlenden k-Raumdaten erst nach der Aufnahme sämtlicher Referenzdatensätze erfolgt.

Werden sogenannte Kernel auf Basis der Referenzdaten gewonnen, um die fehlenden k-Raumdaten für die beschleunigte Bildgebung zu ermitteln, so können mittels eines gleitenden Durchschnitts während einer Aufnahme bzw. Bildgebung mehrere solche aktualisierte Kernel gewonnen werden. Dies hat den Vorteil einer gleichbleibend hohen Bildqualität der Berechnung der fehlenden k-Raumdaten auch bei einem Auftreten der erwähnten dynamischen Störeffekte.

Alternativ kann auch am Ende der Aufnahme ein einziger stabiler Kernel gewonnen werden, indem ein Teil des für die Referenzdaten benötigten k-Raums, beispielsweise das k-Raum-Zentrum, oder ein kompletter Satz der Referenzdaten im Laufe der Messung mehrfach abgetastet bzw. erfasst wird und der Kernel danach auf Basis der gemittelten Referenzdaten berechnet wird.

In einer besonders praktikablen Ausgestaltung des erfindungsgemäßen Verfahrens umfasst das modifizierte erste Abtastschema mindestens vier aufeinanderfolgende Phasenkodiergradienten und mindestens drei aufeinanderfolgende Auslesegradienten mit wechselnder Polarität, welche sich mit den Phasenkodiergradienten zeitlich abwechseln. Vorteilhaft werden mindestens drei unterschiedliche k-Raumzeilen pro Repetition für die Berechnung von k-Raumdaten zur Kompensation der Unterabtastung erfasst, wodurch die Bildaufnahme beschleunigt wird.

Bevorzugt umfassen dabei die zweiten bis vierten Phasenkodiergradienten eine positive Polarität. Bei dieser Variante wird die Abtastrichtung in Phasenkodierrichtung in einer Repetition beibehalten.

Sollen simultan k-Raumdaten in mehreren Schichten aufgenommen werden, so werden bevorzugt mit den zweiten bis vierten Phasenkodiergradienten zeitlich synchronisiert Schichtselektionsgradienten geschaltet. Vorteilhaft kann durch die simultane Akquisition die Aufnahmezeit einer Magnetresonanzbildgebung reduziert werden.

Ganz besonders bevorzugt weisen dabei die Schichtselektionsgradienten eine wechselnde Polarität auf. Dieses Abtastschema ist besonders geeignet für SMS-, GRAPPA + SMS- oder Dual Polarity GRAPPA-Bildgebungsverfahren.

Wird ein Bildgebungsverfahren mit einer Unterabtastung in Phasenkodierrichtung gewählt, beispielsweise ein GRAPPA-Schema oder eine Kombination aus einem GRAPPA-Schema und einem SMS-Schema, so werden die Amplituden der Phasenkodiergradienten des ersten modifizierten Abtastschemas bevorzugt in Abhängigkeit von einem Unterabtastungsfaktor des zweiten Abtastschemas gewählt. Vorzugsweise werden die Amplituden so gewählt, dass bei einem Unterabtastungsfaktor mit dem Wert F (F ist eine natürliche Zahl) F-1 Zeilen übersprungen werden.

In einer bevorzugten Ausgestaltung des erfindungsgemäßen Verfahrens zur Ansteuerung eines Magnetresonanzbildgebungssystems wird für die beschleunigte Echoplanar-Aufnahme eine der folgenden spezifischen Bildgebungstechniken genutzt:
- GRAPPA,
- SMS,
- SENSE,
- eine Kombination aus GRAPPA und SMS,
- eine Aufnahme mit dualer Polarität,
- ein Compressed SENSING- oder SENSE-basiertes Bildgebungsverfahren mit einem Deep Learning-Rekonstruktionsverfahren, was zur Ausführung ebenfalls Spulensensitivitäts-Informationen erfordert.

In Kerstin Hammernik et al., Z-net: Systematic Evaluation of Iterative Deep Neural Networks for Fast Parallel MR Image Reconstruction, https://arxiv.org/abs/1912.09278, ist ein Compressed SENSING- oder SENSE-basiertes Bildgebungsverfahren mit einem Deep Learning-Rekonstruktionsverfahren beschrieben.

Vorteilhaft kann bei der Bildgebung mit einem GRAPPA-Verfahren durch das erste Abtastschema zunächst eine Gewinnung von Nyquist-Ghosting-Korrekturlinien erfolgen und durch Anwendung des ersten modifizierten Abtastschemas zusätzlich eine Gewinnung von Referenzlinien zur GRAPPA-Kalibration, also zur Berechnung von Kerneln zur Ermittlung der eine Unterabtastung ergänzenden k-Raumdaten erfolgen. Durch Modifikation der Phasenkodiergradienten (Gy-Gradienten) erfolgt die Auswahl eines Teilabschnitts des aufzunehmenden k-Raums und mittels zusätzlicher Phasenblips bzw. kleinerer Phasenkodiergradienten erfolgt das Weiterbewegen innerhalb des durch das erste modifizierte Abtastschema gebildeten Navigators. Mit diesem Verfahren lässt sich mit 8 Repetitionen bei einer Erfassung von 3 Phasenkodierlinien pro Repetition mit dem modifizierten ersten Abtastschema bereits ein zur GRAPPA-Kalibration gut geeigneter k-Raum mit 24 Phasenkodierlinien gewinnen. Neben der Kalibration von GRAPPA-Kerneln können die erfassten k-Raumdaten auch zur Kalibration von SENSE-Verfahren und insbesondere zur Gewinnung von Spulensensitivitätskarten für Deep Learning-Rekonstruktionsverfahren genutzt werden.

Vorteilhaft kann bei der Bildgebung mit einem SMS-Verfahren durch das erste Abtastschema eine Gewinnung von Nyquist-Ghosting-Korrekturlinien erfolgen und durch das erste modifizierte Abtastschema eine Gewinnung von Referenzlinien zur SMS-Kalibration, also zur Berechnung von Kerneln zur Ermittlung der eine Unterabtastung ergänzenden k-Raumdaten erfolgen. Vorteilhaft erfolgt so die Gewinnung von Referenzlinien zur SMS-Kernel-Kalibration.

In einer spezifischen Ausgestaltung eines solchen Verfahrens, welches auf der SMS-Abtasttechnik beruht, werden dabei Referenzdaten für die Slice-GRAPPA-Kernel-Berechnung ermittelt. Slice GRAPPA ist das gängige Rekonstruktionsverfahren für SMS-Aufnahmen, siehe **z.B.** https://onlinelibrary.wiley.com/doi/full/10.1002/mrm.23097 Die aufzunehmenden Datenpunkte werden dabei so gewählt bzw. angeordnet, dass ein slice-blip wie eine Verschiebung der Daten in Schichtkodierrichtung (kz-Richtung) behandelt werden kann. Diese Vorgehensweise ist in der US-Patentschrift US 10557903 B2 genauer beschrieben.

Werden in ky-Richtung und kz-Richtung benachbarte k-Raum-punkte durch aufeinanderfolgende ganze Zahlen 1, 2, 3, usw. bezeichnet, so werden bei einer Erfassung von drei Phasenkodierlinien mit dem ersten Abtastschema bei dem modifizierten ersten Abtastschema bei der ersten Repetition die Datenpunkte kz=1/ky=1, kz=2/ky=2, kz=1/ky=3 aufgenommen. Dies erfolgt durch alternierende Blips in kz-Richtung und unipolare Blips in ky-Richtung. Blips sind hier Gradientenpulse, die einen kurzen Trajektorienabschnitt repräsentieren. In der folgenden Repetition wird der komplementäre k-Raum-Abschnitt kz=2/ky=1, kz=1/ky=2, kz=2/ky=3 aufgenommen. Hierbei wird die Polarität der kz-Blips entsprechend gedreht. Anschließend erfolgt die Aufnahme weiterer Teilabschnitte. Alternativ kann die Aufnahme auch "reihenweise" in kz-Richtung erfolgen, d. h. es wird eine einzelne Verschiebung entlang der kz-Achse vor dem ersten Echo vorgenommen und dann ky=1,2,3 mit festen kz-Werten aufgenommen. Wie in der US-Patentschrift US 10557903 B2 beschrieben, können schichtspezifische Kalibrationsdaten dann durch Fourier-Transformation entlang der kz-Achse erzielt werden. Um einen Kernel mit 24 k-Raum-Linien zu erhalten, sind hier bei einer Erfassung von 3 Phasenkodierlinien pro Repetition mit dem ersten Abtastschema 16 Repetitionen erforderlich.

Vorteilhaft kann bei der Bildgebung mit einem kombinierten GRAPPA + SMS-Verfahren durch das erste Abtastschema eine Gewinnung von Nyquist-Ghosting-Korrekturlinien erfolgen und durch das erste modifizierte Abtastschema eine Gewinnung von Referenzlinien zur GRAPPA + SMS-Kalibration, also zur Berechnung von Kerneln zur Ermittlung der eine Unterabtastung ergänzenden k-Raumdaten erfolgen.

Vorteilhaft erfolgt eine Gewinnung von Referenzlinien zur kombinierten GRAPPA + SMS-Kalibration. Auf diese Weise können Referenzdaten sowohl für GRAPPA als auch für SMS gewonnen werden. Gegenüber einer Variante, welche auf ein reines SMS-Bildgebungsverfahren gerichtet ist, ist diese Variante dahingehend modifiziert, dass der Abstand der Phasenkodierlinien in ky-Richtung, d. h. in Phasenkodierrichtung jeweils dem Unterabtastungsfaktor, beispielsweise 2, entspricht. Zur Kalibration der sogenannten Slice-GRAPPA-Kernel werden die erfassten k-Raumdaten dann gemäß DE 10 2017 209 988 B3 so umgeordnet, dass das unterabgetastete Aufnahmeschema beibehalten wird. Zur Kalibration der konventionellen GRAPPA-Kernel werden die Phasenkodierlinien dagegen, wie weiter oben bereits beschrieben, als Block übernommen.

Bei den vorgenannten unterschiedlichen Unterabtastungsverfahren sind auch andere Anordnungen der in den Repetitionen aufgenommenen k-Raum-Punkte denkbar, solange am Ende ein voll abgetasteter k-Raumbereich erzielt wird.

Für eine GRAPPA + SMS-Kalibration sind für zwei Kernel mit je 24 k-Raum-Linien bei einer Erfassung von 3 Phasenkodierlinien pro Repetition mit dem ersten Abtastschema bereits 32 Repetitionen erforderlich.

Umfasst das zweite Abtastschema eine Abtastung mit dualer Polarität, beispielsweise bei einem Dual-Polarity-GRAPPA-Bildgebungsverfahren, so umfasst das erste modifizierte Abtastschema für jede abgetastete Phase mindestens zwei Abtastschemata mit zueinander invertierter Auslesepolarität. Dazu können vorzugsweise zwei aufeinander folgende Repetitionen mit unterschiedlich modifizierten ersten Abtastschemata mit zueinander invertierter Auslesepolarität der Phasenkodiergradienten und ansonsten gleichem Gradientenschema genutzt werden. Im Vergleich zu einem Standard-GRAPPA-Verfahren erhöht sich also bei dieser ersten Untervariante die Zahl der für die Ermittlung der Referenzdaten benötigten Repetitionen um das Doppelte.

Bei einer zweiten Untervariante des Dual-Polarity-GRAPPA-Bildgebungsverfahrens erfolgt die Erfassung der k-Raumdaten mit dem modifizierten ersten Abtastschema mit zueinander invertierter Auslesepolarität, indem jede Phase mit wechselnder Polarität mehrmals, vorzugsweise dreimal abgetastet wird. Bei dieser Untervariante erfolgt also bei der Aufnahme der Referenzdaten pro Repetition nur die Aufnahme einer Phasenkodierlinie, so dass bei einem unmodifizierten ersten Abtastschema mit einer Aufnahme von drei Phasenkodierlinien pro Repetition für eine Erfassung von Nyquist-Ghost-Korrekturdaten die Anzahl der für die Aufnahme der Referenzdaten benötigten Repetitionen verdreifacht wird.

Wird bei dem erfindungsgemäßen Verfahren als beschleunigtes EPI-Bildgebungsverfahren ein Bildgebungsverfahren zum simultanen Auslesen mehrerer Schichten genutzt, wie zum Beispiel die vorgenannten Bildgebungsverfahren, so ist bei einer besonders effektiv realisierbaren Ausgestaltung des Verfahrens die Anzahl N der simultan auszulesenden Schichten gleich 2.

Alternativ kann die Anzahl N der simultan auszulesenden Schichten auch gleich 3 sein. Grundsätzlich wäre es optimal, so viele Schichten wie möglich simultan auszulesen. Allerdings wird die Anzahl der simultan auszulesenden Schichten durch folgende Umstände beschränkt: Die dem Patienten pro Puls zugeführte Energie ist proportional zur Anzahl der Schichten N. Dabei ist die pro Zeit erlaubte Energiezufuhr jedoch limitiert. Außerdem ist die Separation in der Bildrekonstruktion umso schwieriger, je mehr Schichten simultan ausgelesen werden, da das Rauschen in den separierten Bildern ansteigt. Dieser Anstieg verhält sich nichtlinear. Daher ist es in der Praxis nur möglich, einige wenige Schichten simultan auszulesen.

Die Erfindung wird im Folgenden unter Hinweis auf die beigefügten Figuren anhand von Ausführungsbeispielen noch einmal näher erläutert. Es zeigen:
FIG 1 ein Schaubild, welches eine herkömmliche Vorgehensweise für eine Nyquist-Ghost-Korrektur veranschaulicht,
FIG 2 ein Schaubild, welches ein herkömmliches Verfahren zur Ermittlung von B₀-Feldkarten veranschaulicht, bei dem mittels Phasenkodiergradienten in jeder Repetition ein anderer aufzunehmender k-Raum-Abschnitt kodiert wird, der anschließend zu passenden k-Räumen zusammengesetzt wird,
FIG 3 ein Schaubild, das eine Pulssequenzfolge zur Implementierung des in FIG 2 veranschaulichten Bildgebungsverfahrens zeigt,
FIG 4 ein Schema zur Gewinnung von Referenzlinien zur GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung,
FIG 5 ein Schaubild, das eine Pulssequenzfolge zur Implementierung des in FIG 4 veranschaulichten Abtastschemas in einem Echoplanar-Bildgebungsverfahren zeigt,
FIG 6 ein alternatives Schema zur Gewinnung von Referenzlinien zur GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung,
FIG 7 ein Schaubild, das eine Pulssequenzfolge zur Implementierung des in FIG 6 veranschaulichten Abtastschemas in einem Echoplanar-Bildgebungsverfahren zeigt,
FIG 8 ein Schema zur Gewinnung von Referenzlinien zur SMS-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung,
FIG 9 ein Schaubild, das eine Pulssequenzfolge zur Implementierung des in FIG 8 veranschaulichten Abtastschemas in einem Echoplanar-Bildgebungsverfahren zeigt,
FIG 10 ein Schema zur Gewinnung von Referenzlinien zur kombinierten GRAPPA + SMS-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung,
FIG 11 ein Schaubild, das eine Pulssequenzfolge zur Implementierung des in FIG 10 veranschaulichten Abtastschemas in einem Echoplanar-Bildgebungsverfahren zeigt,
FIG 12 ein Schema zur Gewinnung von Referenzlinien eines ersten Kernels für eine Dual-Polarity-GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung,
FIG 13 ein Schaubild, das eine Pulssequenzfolge zur Implementierung des in FIG 12 veranschaulichten Abtastschemas zeigt,
FIG 14 ein Schema zur Gewinnung von Referenzlinien eines zweiten Kernels für eine Dual-Polarity-GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung,
FIG 15 ein Schaubild, das eine Pulssequenzfolge zur Implementierung des in FIG 14 veranschaulichten Abtastschemas zeigt, und
Figur 16 ein Magnetresonanzbildgebungssystem gemäß einem Ausführungsbeispiel der Erfindung.

In FIG 1 ist ein Schaubild 10 mit einem Flussdiagramm, welches eine herkömmliche Vorgehensweise für eine Implementierung einer Nyquist-Ghost-Korrektur in ein bildgebendes Magnetresonanzbildgebungsverfahren veranschaulicht, gezeigt. Dabei werden bei dem Schritt 1.I in den ersten drei Repetitionen REP1, REP2, REP3 drei Phasenkodierlinien im k-Raum-Zentrum im Rahmen einer Phasenkorrekturaufnahme aufgenommen. Die im k-Raumzentrum bei den ersten drei Repetitionen REP1, REP2, REP3 erfassten unkodierten Phasenkodierlinien werden anschließend bei dem Schritt 1.II gemittelt, so dass k-Raumdaten für eine Navigator-Aufnahme NA erhalten werden. Auf Basis dieser gemittelten k-Raumdaten werden bei dem Schritt 1.III Nyquist-Ghost-Effekte korrigiert bzw. aus Bilddaten eliminiert. Dieser Schritt 1.III ist auch durch eine bildliche Darstellung unter dem Flussdiagramm symbolisiert, wobei links im Bild die artefaktbehaftete Bilddarstellung eines Gehirns gezeigt ist und rechts im Bild die korrigierte Bilddarstellung veranschaulicht ist. Bei dieser herkömmlichen Variante werden die Nyquist-Ghost-Korrekturlinien nur in den ersten drei Repetitionen abgetastet und die gewonnenen Korrekturfaktoren werden anschließend auf alle anderen Repetitionen des eigentlichen Bildgebungsverfahrens angewendet. Die in FIG 1 veranschaulichte Methode zur Korrektur von Nyquist-Ghosteffekten ist in US 604 365 1 B2 ausführlich erläutert.

Es soll an dieser Stelle erwähnt werden, dass es auch herkömmlich üblich ist, in jeder Repetition vor der Abtastung der k-Raumdaten für die Bildgebung jeweils drei unkodierte k-Raumlinien mit wechselnder Polarität als Navigatordaten abzutasten. Die in FIG 1 beschriebene Variante ist an die in FIG 2 beschriebene Gewinnung von phasenkodierten k-Raumlinien zur Gewinnung von B₀-Feldkarten angepasst. Die in FIG 2 veranschaulichten Repetitionen REP4, **...,** REPM schließen sich in diesem speziellen Fall an die in FIG 1 veranschaulichten ersten drei Repetitionen REP1, REP2, REP3 an.

In FIG 2 ist ein Schaubild 20 gezeigt, welches ein herkömmliches Verfahren zur bildbasierten Ermittlung von Feldkarten veranschaulicht. Das in FIG 2 veranschaulichte Verfahren entspricht der Vorgehensweise in Chi et al., "Field-Mapping-Embedded EPI for Geometric Distortion Correction", Proc. Intl. Soc. Mag. Reson. Med. 30 (2022) 1099.

Bei diesem Verfahren wird mittels Phasenkodiergradienten in jeder Repetition ein anderer aufzunehmender k-Raum-Abschnitt kodiert, der anschließend zu passenden k-Räumen zusammengesetzt wird (siehe Schritte 2.I, 2.II). Bei dem Schritt 2.I erfolgen eine Reihe von Repetitionen REP4, REP5, **...,** REPM, bei denen k-Raumdaten bzw. Rohdaten mit unterschiedlicher Phasenkodierung im Rahmen einer Echoplanar-Bildgebung von einem Untersuchungsobjekt schichtweise erfasst werden. Anders als bei dem in FIG 1 veranschaulichten Verfahren wird neben unterabgetasteten k-Raumdaten für die Bildgebung bei jeder Repetition auch eine Phasenkodierlinie für die Erstellung einer späteren Feldkarte aufgenommen, wobei für jede Repetition eine andere Phase kodiert wird. Bei dem Schritt 2.II werden die bei dem Schritt 2.I erfassten unterabgetasteten k-Raumlinien umgeordnet. Außerdem erfolgt bei dem Schritt 2.II eine zusätzliche Aufnahme von Referenzdaten REF, welche später für eine Kalibration von Kerneln für eine Ermittlung von bei der Aufnahme der k-Raumdaten bei dem Schritt 2.I fehlenden k-Raumdaten genutzt werden. Es soll an dieser Stelle ausdrücklich darauf hingewiesen werden, dass die Aufnahme der Referenzdaten in einer gesonderten Aufnahme erfolgt. Es werden also zwei separate Aufnahmen, eine Aufnahme für die Aufnahme der unterabgetasteten k-Raumdaten und der Phasenkodierlinien und eine separate Aufnahme der Referenzdaten REF durchgeführt.

Anschließend erfolgt bei dem Schritt 2.III eine Ermittlung der fehlenden k-Raumdaten für die jeweiligen Schichten auf Basis der bei dem Schritt 2.II ermittelten Kernel und der unterabgetasteten k-Raumdaten.

Bei dem Schritt 2.IV erfolgen schichtweise Bildrekonstruktionen mit Hilfe einer zweidimensionalen schnellen Fourier-Transformation (2D-FFT) auf Basis der vervollständigten k-Raumdaten.

Bei dem Schritt 2.V erfolgt auf Basis der Mehrzahl von rekonstruierten Schichtbildern eine Berechnung einer Phasendifferenz (beispielsweise die Phasenentwicklung zwischen dem ersten und dritten Echo Δϕ₃₁, welche mit gleicher Polarität aufgenommen werden) und eines Gesamtphasenoffsets ϕ₀.

Anschließend werden bei dem Schritt 2.VI die Bilddaten der einzelnen Kanäle kombiniert und bei dem Schritt 2.VII erfolgt ein Auswickeln (im Englischen "Unwrapping") von Phasen zur Erstellung der Feldkarten. Damit erfolgt ein Beseitigen von Phasensprüngen im Bild. Dadurch dass die Phase ja mit 360 Grad periodisch ist, kann es zwischen Pixeln zu Uneindeutigkeiten kommen (d.h. eine Phasendifferenz wird zu 0, die benachbarte zu 360°).

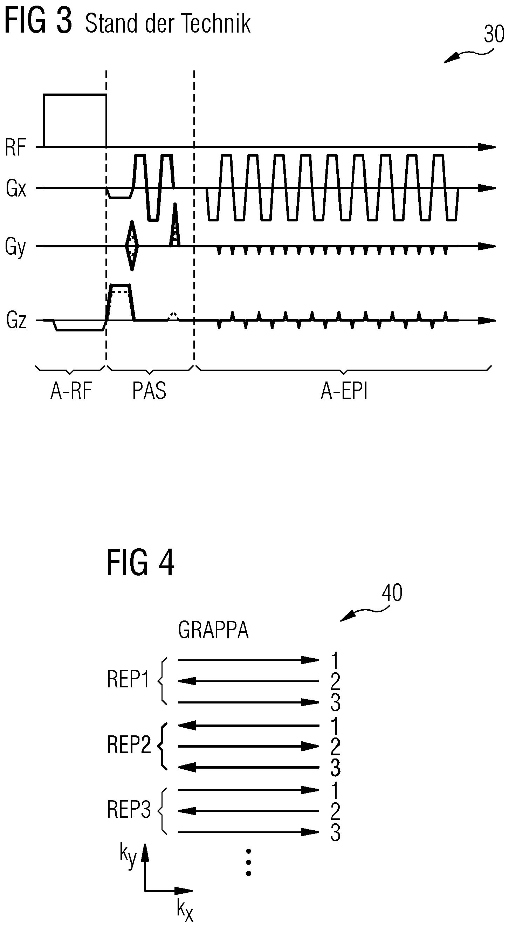

In FIG 3 ist ein Schaubild 30, das eine Pulssequenzfolge zur Implementierung des in FIG 2 veranschaulichten Verfahrens zur bildbasierten Ermittlung von Feldkarten veranschaulicht, gezeigt. Das Schaubild umfasst vier untereinander dargestellte Teilschaubilder, wobei das erste Teilschaubild einen Anregungshochfrequenzpuls RF zeigt, das zweite Teilschaubild eine Serie von Auslesegradienten Gx, das dritte Teilschaubild eine Serie von Phasenkodiergradienten Gy und das vierte Teilschaubild eine Serie von Schichtselektionsgradienten Gz darstellt. Es soll an dieser Stelle angemerkt erden, dass bei dieser Darstellung die empfangenen Hochfrequenzpulse, welche die empfangenen Messsignale repräsentieren, nicht veranschaulicht sind.

Links im Schaubild ist ein Anregungsschema A-RF mit einem HF-Anregungspuls RF und einem simultan dazu ausgespielten Schichtselektionsgradienten Gz gezeigt.

In der Mitte im Schaubild ist zwischen zwei vertikal verlaufenden gestrichelten Linien ein Abtastschema PAS zur Abtastung von phasenverschobenen Phasenkodierlinien für eine Navigator-Aufnahme NA zur Akquisition von k-Raumdaten für eine Nyquist-Ghosting-Korrektur dargestellt. Für die Positionierung einer gewünschten Ausgangsposition im k-Raum werden zunächst simultan ein Frequenzkodiergradient Gx, ein Schichtselektionsgradient Gz und ein Phasenkodiergradient Gy geschaltet. Anschließend werden drei hinsichtlich ihrer Polarität alternierende Frequenzkodiergradienten Gx geschaltet. Im Rahmen der Erzeugung der Phasenkorrekturdaten wird mittels eines Wechsels der Polarität der Auslesegradienten auf der Achse in Richtung des Auslesegradienten Gx die Polarität der Auslesegradienten in kx-Richtung gespiegelt, was für die Gewinnung von drei unipolaren k-Räumen für die spätere bildbasierte Berechnung der Feldkarten erforderlich ist.

Zum Abschluss des mittleren Abtastschemas PAS wird ein Phasenkodiergradient Gy und ein Schichtkodiergradient Gz geschaltet.

Rechts davon abgesetzt ist eine EPI-Pulssequenz EPI mit einem Slice-GRAPPA-Abtastschema dargestellt.

In FIG 4 ist ein Schema 40 zur Gewinnung von Referenzlinien zur GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer GRAPPA-Unterabtastung erfasst werden, gemäß einem Ausführungsbeispiel der Erfindung dargestellt.

Wie bereits im allgemeinen Teil ausführlicher erläutert, wird im Rahmen des erfindungsgemäßen Verfahrens ein erstes Abtastschema (nicht gezeigt) für die unkodierte Aufnahme von drei Phasenkodierlinien für eine Korrektur von Nyquist-Ghost-Effekten verwendet und eine entsprechende Plussequenz in einer ersten Repetition im Rahmen eines Bildgebungsvorgangs ausgespielt. Für die folgenden Repetitionen wird nun das Abtastschema, wie in FIG 4 dargestellt, modifiziert.

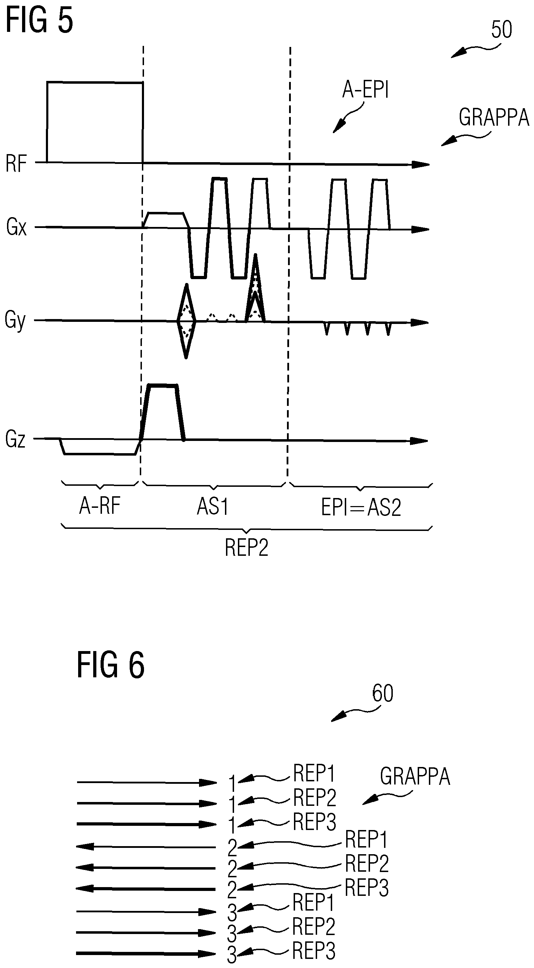

Hierfür wird das erste Aufnahmeschema zur Gewinnung von Referenzlinien für Kernel, welche für eine Ermittlung von fehlenden Daten aufgrund der Unterabtastung bei dem GRAPPA-Bildgebungsverfahren benötigt werden, dahingehend modifiziert, dass zusätzliche sogenannte Phasenblips oder Phasenkodiergradienten hinzugefügt werden. Durch die bereits in FIG 3 gezeigten Phasenkodiergradienten bzw. Gy-Gradienten erfolgt die Auswahl eines Teilabschnitts REP1, REP2, REP3 des aufzunehmenden k-Raums und mittels zusätzlicher Phasenblips, die in FIG 5 veranschaulicht sind, erfolgt ein Weiterbewegen zwischen den einzelnen Phasenkodierzeilen einer Repetition. Mit diesem Verfahren lässt sich mit acht Repetitionen bereits ein zur GRAPPA-Kalibration gut geeigneter k-Raum mit 24 Phasenkodierlinien gewinnen.

In FIG 5 ist ein Schaubild 50 gezeigt, das eine Pulssequenzfolge zur Implementierung des in FIG 4 veranschaulichten Abtastschemas in einem Echoplanar-Bildgebungsverfahren zeigt. Zunächst ist in dem in FIG 5 veranschaulichten Schaubild 50 das bereits in FIG 3 veranschaulichte Anregungsschema A-RF dargestellt. Das darauffolgende, zwischen gestrichelten vertikalen Linien angeordnete, modifizierte erste Abtastschema AS1 spiegelt die in FIG 4 dargestellte zweite Repetition REP2 wider, mit der Referenzdaten erfasst werden sollen. Zur Positionierung am Startpunkt der in FIG 4 dargestellten Trajektorie der zweiten Repetition REP2 werden zunächst simultan ein Frequenzkodiergradient Gx, ein Schichtselektionsgradient Gz und ein Phasenkodiergradient Gy ausgespielt. Die mit unterschiedlicher Amplitude (einmal dick und einmal gestrichelt) übereinander an derselben Stelle gezeichneten Phasenkodiergradienten Gy sind dabei unterschiedlichen Repetitionen zugeordnet. Je nach Amplitudenstärke wird bei unterschiedlichen Repetitionen eine unterschiedliche Phase im k-Raum angesteuert. Anschließend folgen ähnlich wie bei der in FIG 3 dargestellten Pulssequenz drei Frequenzkodiergradienten Gx mit wechselnder Polarität, denen jeweils ein als Phasenblip bezeichneter kleiner zweiter bzw. dritter Phasenkodiergradient Gy folgt. Schließlich wird das modifizierte erste Anregungsschema AS1 durch einen zusätzlichen Frequenzkodiergradienten Gx und einen dazu simultanen vierten Phasenkodiergradienten Gy abgeschlossen, um zum Ausgangspunkt der den Echozug der eigentlichen EPI-Pulssequenz EPI für die Echoplanar-Bildgebung A-EPI bildenden Trajektorie zu kommen. Auch hier ist der das modifizierte erste Abtastschema abschließende Phasenkodiergradient Gy wieder mit einer Mehrzahl von übereinander gezeichneten Phasenkodiergradienten Gy gezeichnet, welche jeweils unterschiedliche Amplituden aufweisen und unterschiedlichen Repetitionen zugeordnet sind. Die EPI-Pulssequenz EPI repräsentiert ein zweites Abtastschema AS2 und weist ähnlich wie das durch die vergrößerten Phasenkodiergradienten Gy modifizierte erste Abtastschema AS1 einheitlich, allerdings negativ polarisierte Phasenkodiergradienten Gy auf.

Es ist anzumerken, dass bei dem in FIG 5 veranschaulichten Ausführungsbeispiel den auch in FIG 4 gezeigten Repetitionen REP1, ..., REP3 eine unmodifizierte Repetition (nicht gezeigt) vorausgeht, mit der drei unkodierte Phasenraumlinien mit wechselnder Polarität als Navigatordaten für eine Nyquist-Ghost-Korrektur aufgezeichnet werden. Bei dieser vorausgehenden Repetition fallen die in FIG 5 im ersten Abtastschema gezeichneten Phasenkodiergradienten, insbesondere die zweiten und dritten Phasenkodiergradienten, auch als Phasenblips bezeichnet, weg, da die Aufnahme der Navigatordaten unkodiert erfolgt. Im Gegensatz zu dem in FIG 2 veranschaulichten herkömmlichen Verfahren erfolgt also die Akquisition der Navigatordaten, der Referenzdaten und der k-Raumdaten für die Echoplanar-Bildgebung mit einer einzigen Pulssequenz und einer gemeinsamen Aufnahme. Wie bereits vorstehend erwähnt, lässt sich durch die Integration der Akquisition der Referenzdaten in den Aufnahmeprozess der Echoplanar-Bildgebung die Gesamtaufnahmezeit reduzieren.

In FIG 6 ist ein alternatives Schema 60 zur Gewinnung von Referenzlinien zur GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung dargestellt. Anders als bei dem in FIG 4 gezeigten Schema 40 sind die aufeinanderfolgenden Phasenkodierlinien einer Repetition REP1, REP2, REP3 jeweils um drei Phasenkodierlinien versetzt angeordnet.

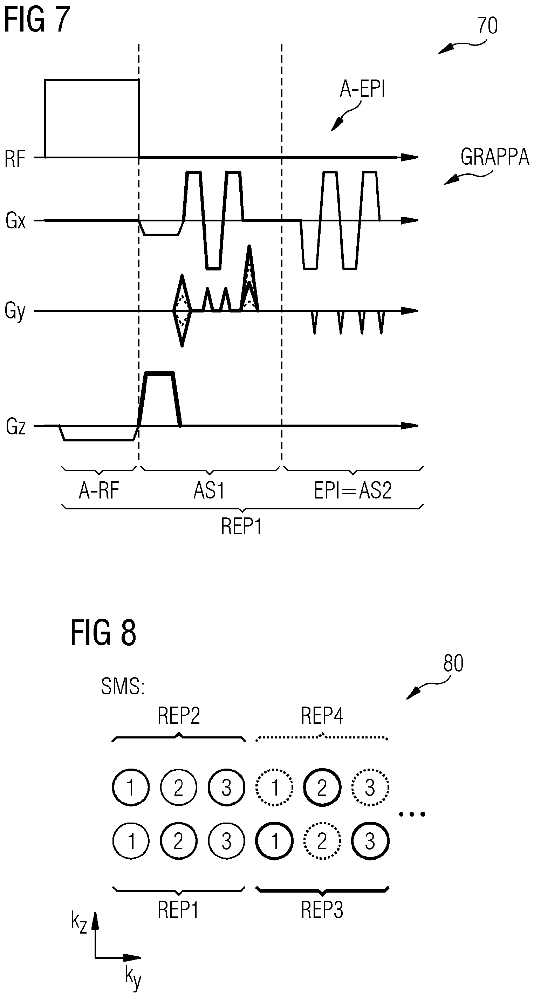

In FIG 7 ist ein Schaubild 70 mit einer Pulssequenzfolge zur Implementierung des in FIG 6 veranschaulichten Abtastschemas in einem Echoplanar-Bildgebungsverfahren gezeigt.

In FIG 7 ist zunächst das bereits in FIG 3 und FIG 5 veranschaulichte Anregungsschema A-RF dargestellt. Das darauffolgende modifizierte erste Abtastschema AS1 spiegelt die in FIG 6 dargestellte erste Repetition REP1 wider. Zur Positionierung am Startpunkt der in FIG 6 dargestellten Trajektorie der ersten Repetition REP1 werden zunächst simultan ein Frequenzkodiergradient Gx, ein Schichtselektionsgradient Gz und ein Phasenkodiergradient Gy ausgespielt. Anschließend folgen ähnlich wie bei der in FIG 5 dargestellten Pulssequenz drei Frequenzkodiergradienten Gx mit wechselnder Polarität, wobei den ersten beiden Frequenzkodiergradienten Gx jeweils ein als Phasenblip bezeichneter Phasenkodiergradient Gy folgt. Dieser Phasenkodiergradient weist eine im Vergleich zu den entsprechenden in FIG 5 gezeigten Phasenkodiergradienten Gy eine dreimal so große Fläche auf, da sie dazu genutzt werden, zwei benachbarte Phasenkodierlinien zu überspringen. Schließlich wird das modifizierte erste Anregungsschema AS1 durch einen zusätzlichen Phasenkodiergradienten abgeschlossen, um zum Ausgangspunkt der den Echozug der eigentlichen EPI-Pulssequenz EPI bildenden Trajektorie zu kommen. Die EPI-Pulssequenz EPI repräsentiert ein zweites Abtastschema AS2 und weist ähnlich wie das durch die vergrößerten Phasenkodiergradienten Gy modifizierte erste Abtastschema AS1 einheitlich, allerdings negativ polarisierte Phasenkodiergradienten auf.

In FIG 8 ist ein Schema 80 zur Gewinnung von Referenzlinien zur SMS-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer SMS-Unterabtastung erfasst werden, gemäß einem Ausführungsbeispiel der Erfindung dargestellt.

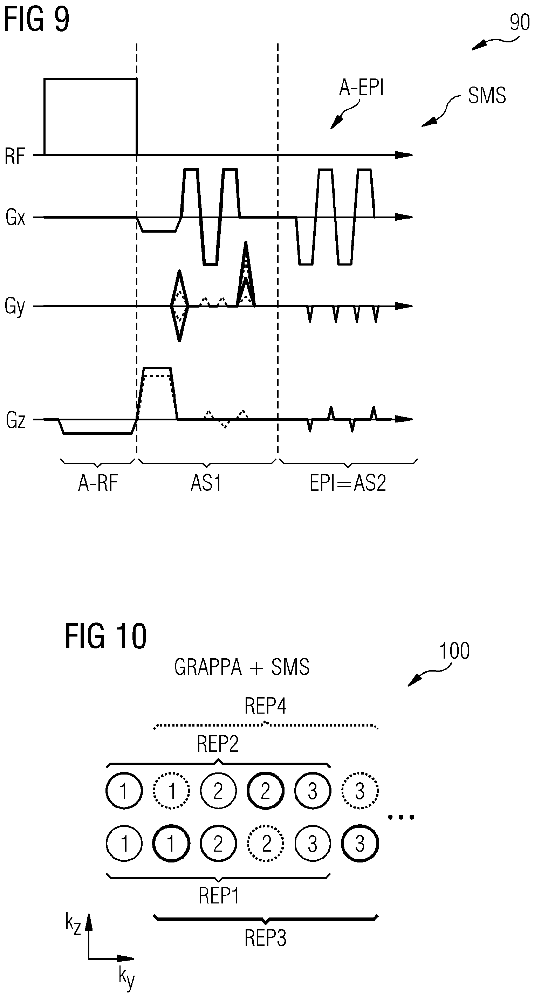

Es werden Referenzdaten für die Slice-GRAPPA-Kernel-Berechnung bei einer SMS-Bildgebung gezeigt. Die aufzunehmenden Datenpunkte sind hier so dargestellt, dass ein slice-blip Gz wie eine Verschiebung der Daten in kz-Richtung behandelt werden kann (vgl. US 10557903 B2). In der ersten Repetition REP1 werden somit die Datenpunkte kz=1/ky=1, kz=2/ky=2, kz=1/ky=3 aufgenommen. Die Reihenfolge der Abtastung der unterschiedlichen k-Raumlinien innerhalb einer Repetition ist mit den diesen k-Raumlinien zugeordneten Zahlen 1, 2, 3 symbolisiert. Dies erfolgt in FIG 9 durch alternierende Blips Gz in kz-Richtung und unipolare Blips Gy in ky-Richtung.

In der zweiten Repetition REP2 wird der komplementäre k-Raum-Abschnitt kz=2/ky=1, kz=1/ky=2, kz=2/ky=3 aufgenommen. Hierbei wird die Polarität der kz-Blips Gz entsprechend gedreht. Anschließend erfolgt die Aufnahme weiterer Teilabschnitte des k-Raums mit der dritten Repetition REP 3 und der vierten Repetition REP4.

Alternativ kann die Aufnahme auch "reihenweise" in kz-Richtung erfolgen, d. h. es wird eine einzelne Verschiebung entlang kz vor dem ersten Echo vorgenommen und dann ky=1,2,3 mit festen kz aufgenommen. Wie in US 10557903 B2 beschrieben, können schichtspezifische Kalibrationsdaten dann durch Fourier-Transformation entlang kz erzielt werden. Um einen Kernel mit 24 k-Raum-Linien zu erhalten, sind hier 16 Repetitionen erforderlich.

In FIG 9 ist ein Schaubild 90 mit einer Pulssequenzfolge zur Implementierung des in FIG 8 veranschaulichten Abtastschemas in einem Echoplanar-Bildgebungsverfahren gezeigt.

In FIG 9 ist zunächst das bereits in FIG 3, FIG 5 und FIG 7 veranschaulichte Anregungsschema A-RF dargestellt. Das darauf folgende modifizierte erste Abtastschema AS1 spiegelt die in FIG 8 dargestellte erste Repetition REP1 wider. Zur Positionierung am Startpunkt der in FIG 8 dargestellten Trajektorie der ersten Repetition REP1 werden zunächst simultan ein Frequenzkodiergradient Gx, ein Schichtselektionsgradient Gz und ein Phasenkodiergradient Gy ausgespielt. Anschließend folgen ähnlich wie bei der in FIG 7 dargestellten Pulssequenz drei Frequenzkodiergradienten Gx mit wechselnder Polarität, wobei den ersten beiden Frequenzkodiergradienten Gx jeweils ein als Phasenblip bezeichneter positiv polarisierter Phasenkodiergradient Gy folgt. Dieser Phasenkodiergradient Gy weist eine im Vergleich zu den entsprechenden in FIG 7 gezeigten Phasenkodiergradienten Gy nur ein Drittel so kleine Fläche auf, da sie dazu genutzt werden, benachbarte Phasenkodierlinien anzusteuern. Zeitlich simultan mit den als Phasenblip bezeichneten Phasenkodiergradienten Gy werden alternierend polarisierte Schichtselektionsgradienten Gz geschaltet.

Schließlich wird das modifizierte erste Anregungsschema AS1 durch einen zusätzlichen Phasenkodiergradienten Gy, mit dem simultan ein letzter Schichtselektionsgradient Gz geschaltet wird, abgeschlossen, um zum Ausgangspunkt der den Echozug der eigentlichen EPI-Pulssequenz EPI bildenden Trajektorie zu kommen. Die EPI-Pulssequenz EPI repräsentiert ein zweites **Ab**tastschema AS2 und weist ähnlich wie das modifizierte erste Abtastschema AS1 ein durch einheitlich, allerdings negativ polarisierte Phasenkodiergradienten Gy und ihre Polarisation wechselnde Schichtselektionsgradienten Gz gekennzeichnetes modifiziertes Abtastschema auf.

In FIG 10 ist ein Schema 100 zur Gewinnung von Referenzlinien zur kombinierten GRAPPA + SMS-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung gezeigt.

Mit dem in FIG 10 veranschaulichten Verfahren können Referenzdaten sowohl für GRAPPA als auch für SMS gewonnen werden. Gegenüber dem in FIG 8 veranschaulichten Abtastschema 80 ist das Verfahren dahingehend modifiziert, dass der Abstand in ky-Richtung jeweils dem Unterabtastungsfaktor (hier 2) entspricht. Das Muster der Linien (gepunktet, dünn, mittel, dick) der den einzelnen Repetitionen REP1, ..., REP4 zugeordneten Klammern entspricht dem Muster der Umfangslinien der den jeweiligen Repetitionen zugeordneten k-Raumlinien. Zur Kalibration der Slice-GRAPPA-Kernel werden die Aufnahmedaten dann gemäß DE 10 2017 209 988 B3 so umgeordnet, dass das unterabgetastete Aufnahmeschema beibehalten wird. Zur Kalibration der konventionellen GRAPPA-Kernel werden die Linien als Block übernommen. Wie bei dem in FIG 8 veranschaulichten Verfahren sind hier auch andere Anordnungen der in den Repetitionen REP1, REP2, REP3, REP4 aufgenommenen k-Raum-Punkte denkbar, solange am Ende ein voll abgetasteter Bereich erzielt wird. Hier sind für zwei Kernel mit je 24 k-Raum-Linien bereits 32 Repetitionen erforderlich.

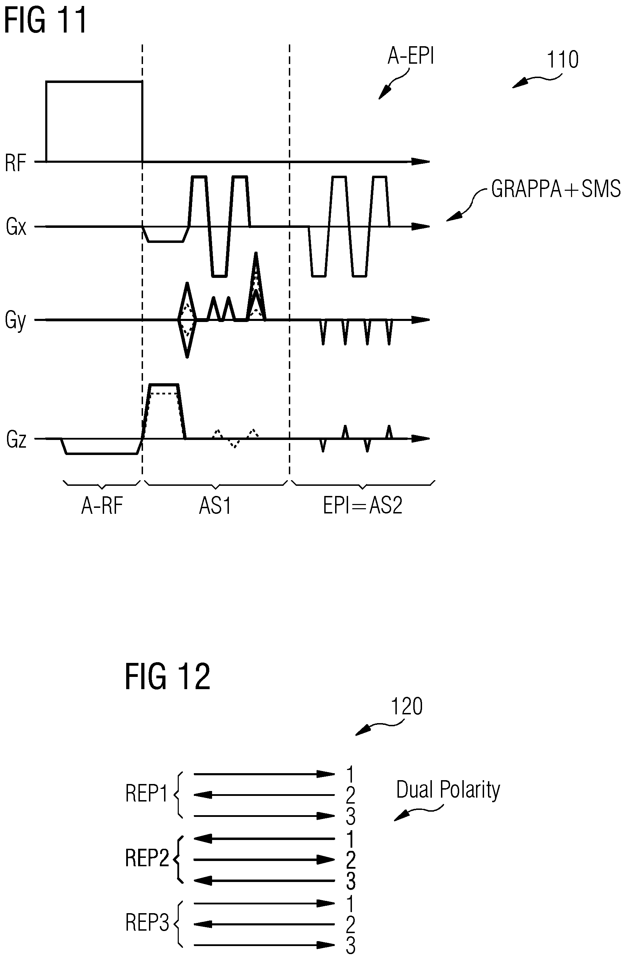

In FIG 11 ist ein Schaubild 110, das eine Pulssequenzfolge zur Implementierung des in FIG 10 veranschaulichten Bildgebungsverfahrens zeigt, veranschaulicht.

In FIG 11 ist zunächst das bereits in FIG 3, FIG 5, FIG 7 und FIG 9 bereits veranschaulichte Anregungsschema A-RF dargestellt. Das darauffolgende modifizierte erste Abtastschema AS1 spiegelt die in FIG 10 dargestellte erste Repetition REP1 wider. Zur Positionierung am Startpunkt der in FIG 10 dargestellten Trajektorie der ersten Repetition REP1 werden zunächst simultan ein Frequenzkodiergradient Gx, ein Schichtselektionsgradient Gz und ein Phasenkodiergradient Gy ausgespielt. Anschließend folgen ähnlich wie bei der in FIG 9 dargestellten Pulssequenz drei Frequenzkodiergradienten Gx mit wechselnder Polarität, wobei den ersten beiden Frequenzkodiergradienten Gx jeweils ein als Phasenblip bezeichneter positiv polarisierter Phasenkodiergradient Gy folgt. Dieser Phasenkodiergradient weist eine im Vergleich zu den entsprechenden in FIG 9 gezeigten Phasenkodiergradienten Gy verdoppelte Fläche auf, da sie dazu genutzt werden, in ky-Richtung jeweils eine Phasenkodierlinie zu überspringen und die übernächste Phasenkodierlinie anzusteuern. Zeitlich simultan mit den als Phasenblip bezeichneten Phasenkodiergradienten Gy werden wie in FIG 9 alternierend polarisierte Schichtselektionsgradienten Gz geschaltet.

Schließlich wird das modifizierte erste Anregungsschema AS1 durch einen zusätzlichen Phasenkodiergradienten Gy, mit dem simultan ein letzter Schichtselektionsgradient Gz geschaltet wird, abgeschlossen, um zum Ausgangspunkt der den Echozug der eigentlichen EPI-Pulssequenz EPI bildenden Trajektorie zu kommen. Die EPI-Pulssequenz EPI repräsentiert ein zweites Abtastschema AS2 und weist ähnlich wie das modifizierte erste Abtastschema AS1 ein durch einheitlich, allerdings negativ polarisierte Phasenkodiergradienten Gy und ihre Polarisation wechselnde Schichtselektionsgradienten Gz gekennzeichnetes modifiziertes Abtastschema auf.

In FIG 12 ist ein Schema 120 zur Gewinnung von Referenzlinien eines ersten Kernels für eine Dual-Polarity-GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung gezeigt. Die Abtastung des k-Raums für den ersten Kernel erfolgt sehr analog zu dem in FIG 4 gezeigten Abtastschema 40 und wird daher nicht nochmals ausführlich erläutert.

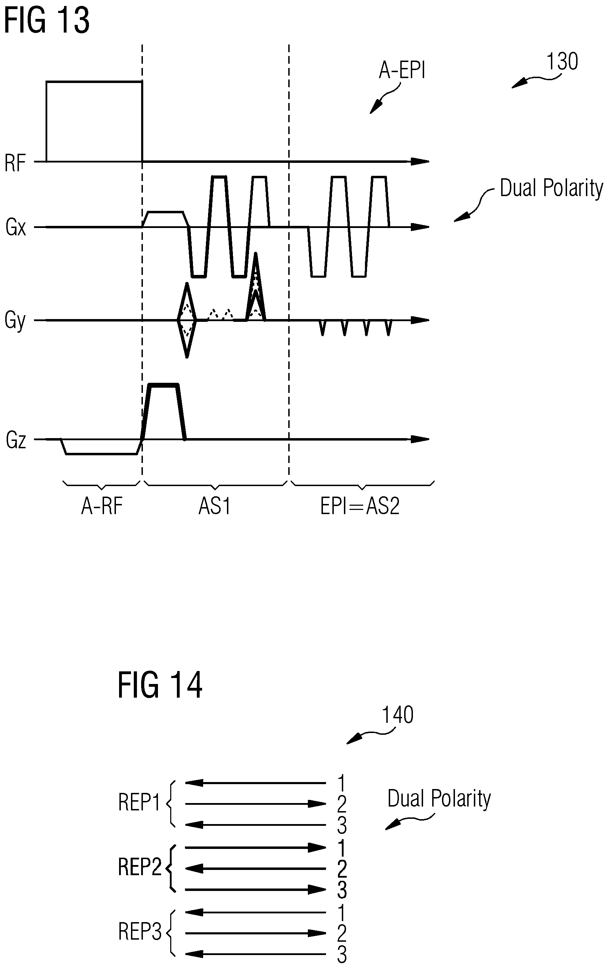

In FIG 13 ist ein Schaubild 130, das eine Pulssequenzfolge zur Implementierung des in FIG 12 veranschaulichten Abtastschemas 120 zeigt, dargestellt. Die in FIG 13 dargestellte Pulssequenz ist analog bzw. identisch zu der in FIG 5 dargestellten Pulssequenz 50 strukturiert und wird daher nicht nochmals ausführlich erläutert.

In FIG 14 ist ein Schema 140 zur Gewinnung von Referenzlinien eines zweiten Kernels für eine Dual Polarity GRAPPA-Kalibration bei einem Verfahren zur Ansteuerung eines Magnetresonanzbildgebungssystems zur Erzeugung von Magnetresonanzbilddaten eines Untersuchungsobjekts, bei dem k-Raumdaten im Rahmen einer beschleunigten Echoplanar-Bildgebung mit einer Unterabtastung erfasst werden, gemäß einem Ausführungsbeispiel der Erfindung gezeigt. Die Abtastrichtung der Phasenkodierlinien erfolgt in FIG 14 gerade umgekehrt zu dem in FIG 12 gezeigten Abtastschema 120.

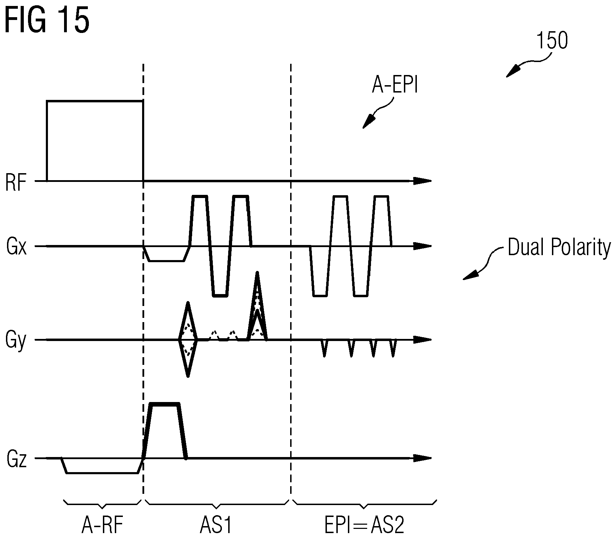

In FIG 15 ist ein Schaubild 150, das eine Pulssequenzfolge des in FIG 14 veranschaulichten Abtastschemas zeigt, dargestellt. Die in FIG 15 dargestellte Pulssequenz unterscheidet sich von der in FIG 13 dargestellten Pulssequenz nur dadurch, dass die Auslesegradienten Gx des modifizierten Abtastschemas AS1 eine im Vergleich zur FIG 13 umgekehrte Polarität aufweisen und ein vierter Auslesegradient Gx in dem modifizierten ersten Abtastschema AS1 entfällt.

Wie von Chi et al., "Field-Mapping-Embedded EPI for Geometric Distortion Correction", Proc. Intl. Soc. Mag. Reson. Med. 30 (2022) 1099, beschrieben, sollte bei den veranschaulichten Aufnahmeschemata zunächst vorzugsweise vorab mindestens eine unkodierte Navigator-Aufnahme bzw. eine Aufnahme für eine Nyquist-Ghost-Korrektur mit einem ersten unmodifizierten Abtastschema vorgenommen werden, um weiterhin eine Ghosting-Korrektur zu ermöglichen. Dazu eignet sich auch eine Kombination mit den bereits angesprochenen Ghosting-Korrektur-Verfahren für eine SMS-Bildgebung (siehe US 10162037 B2 bzw. US 11280870 B2). Wird nur eine solche Aufnahme für Ghosting-Korrekturen durchgeführt, erfolgt wie beim herkömmlichen "externen Phasenkorrekturscan" die Korrektur statisch, d. h. dynamische Veränderungen können nicht einfließen. Da Drifteffekte sich normalerweise zeitlich langsam entwickeln, ist das Einstreuen dieser Aufnahmen z. B. alle acht Repetitionen und ein Update der Ghosting-Korrekturfaktoren z. B. mittels gleitenden Durchschnitts, effektiv, um diese langsamen Drifteffekte zu kompensieren.

Wie bereits erläutert, ist es ebenfalls denkbar, bei sehr langen Bildaufnahmen, z. B. mittels gleitenden Durchschnitts, mehrere GRAPPA- und/oder SMS-Kernel zu gewinnen. Dies hat den Vorteil einer gleichbleibend hohen Bildqualität auch bei Störeinflüssen wie Phasenänderungen oder Patientenbewegungen.

Alternativ kann ein stabiler Kernel gewonnen werden, indem ein Teil (z. B. das k-Raum-Zentrum) oder der komplette Kalibrations-k-Raum im Laufe der Messung mehrfach abgetastet wird und die Kalibration auf allen gemittelten Daten erfolgt.

Die in der Figurenbeschreibung skizzierten Aufnahmeschemata wurden basierend auf einer aus drei Linien bestehenden Navigator-Aufnahme beschrieben. Dies hat den Vorteil, dass sich durch die Modifikation keine Veränderung von Repetitionszeit TR und Echozeit TE ergibt. Jedoch ist eine Mindestanzahl an Repetitionen erforderlich, um vollständige Datensätze zu gewinnen. Natürlich ist es auch denkbar, die Anzahl der Navigatorlinien unter Inkaufnahme einer leichten Zunahme der Echozeit TE und potentiell der Repetitionszeit TR zu erhöhen, um mehr Referenzlinien je Repetition zu gewinnen.

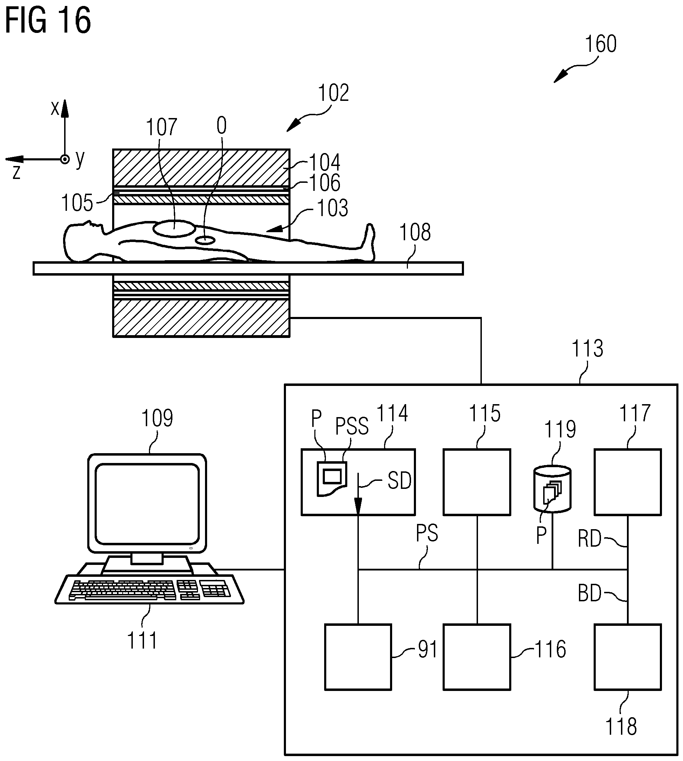

In FIG 16 ist grob schematisch eine erfindungsgemäße Magnetresonanzanlage 160 (im Folgenden kurz "MR-Anlage" genannt) dargestellt. Sie umfasst zum einen den eigentlichen Magnetresonanzscanner 102 mit einem Untersuchungsraum 103 bzw. Patiententunnel, in den auf einer Liege 108 ein Patient bzw. Untersuchungsobjekt O, in dessen Körper sich beispielsweise ein bestimmtes abzubildendes Organ befindet, eingefahren werden kann.

Der Magnetresonanzscanner 102 ist in üblicher Weise mit einem Grundfeldmagnetsystem 104, einem Gradientensystem 106 sowie einem HF-Sendeantennensystem 105 und einem HF-Empfangsantennensystem 107 ausgestattet. In dem dargestellten Ausführungsbeispiel handelt es sich bei dem HF-Sendeantennensystem 105 um eine im Magnetresonanzscanner 102 fest eingebaute Ganzkörperspule, wogegen das HF-Empfangsantennensystem 107 aus am Patienten bzw. Probanden anzuordnenden Lokalspulen besteht (in FIG 16 nur durch eine einzelne Lokalspule symbolisiert). Grundsätzlich kann aber auch die Ganzkörperspule des HF-Sendeantennensystems 105 als HF-Empfangsantennensystem genutzt werden und die Lokalspulen 107 als HF-Sendeantennensystem, sofern diese Spulen jeweils in unterschiedliche Betriebsweisen umschaltbar sind.

Die MR-Anlage 160 weist weiterhin eine zentrale Steuereinrichtung 113 auf, die zur Steuerung der MR-Anlage 160 verwendet wird. Diese zentrale Steuereinrichtung 113 umfasst eine Sequenzsteuereinheit 114 zur Pulssequenzsteuerung. Mit dieser wird die zeitliche Abfolge von Hochfrequenz-Pulsen (HF-Pulsen) und von Gradientenpulsen in Abhängigkeit von einer gewählten Bildgebungssequenz PS gemäß einem Pulssequenzschema PSS gesteuert. Eine solche Bildgebungssequenz PS bzw. das der Bildgebungssequenz PS zugrundeliegende Pulssequenzschema PSS kann beispielsweise innerhalb eines Mess- oder Steuerprotokolls P vorgegeben sein. Üblicherweise sind verschiedene Steuerprotokolle P für unterschiedliche Messungen in einem Speicher 119 hinterlegt und können von einem Bediener ausgewählt (und bei Bedarf gegebenenfalls geändert) und dann zur Durchführung der Messung genutzt werden. Die Sequenzsteuereinheit 114 umfasst auch eine erfindungsgemäße Ansteuersequenz-Ermittlungseinrichtung 91. Die Ansteuersequenz-Ermittlungseinrichtung 91 erzeugt Steuerdaten SD, welche die in FIG 5, FIG 7, FIG 9, FIG 11, FIG 13 oder FIG 15 gezeigte Reihenfolge von Anregungsmodulen und Auslesemodulen ermöglichen, die die Sequenzsteuereinheit 114 zur Pulssequenzsteuerung ausgibt.

Zur Ausgabe der einzelnen HF-Pulse weist die zentrale Steuereinrichtung 113 eine Hochfrequenzsendeeinrichtung 115 auf, die die HF-Pulse erzeugt, verstärkt und über eine geeignete Schnittstelle (nicht im Detail dargestellt) in das HF-Sendeantennensystem 105 einspeist. Zur Steuerung der Gradientenspulen des Gradientensystems 106 weist die Steuereinrichtung 113 eine Gradientensystemschnittstelle 116 auf. Die Sequenzsteuereinheit 114 kommuniziert in geeigneter Weise, z. B. durch Aussendung von Sequenzsteuerdaten SD, mit der Hochfrequenzsendeeinrichtung 115 und der Gradientensystemschnittstelle 116 zur Aussendung der Pulssequenzen PS in der durch die Ansteuersequenz-Ermittlungseinrichtung 91 erzeugten Reihenfolge. Die Steuereinrichtung 113 weist außerdem eine (ebenfalls mit der in geeigneter Weise mit der Sequenzsteuereinheit 114 kommunizierende) Hochfrequenzempfangseinrichtung 117 auf, um koordiniert vom HF-Sendeantennensystem 107 empfangene Magnetresonanz-Signale zu akquirieren. Eine Rekonstruktionseinheit 118 übernimmt die akquirierten Daten nach Demodulierung und Digitalisierung als Rohdaten bzw. k-Raumdaten RD und rekonstruiert daraus die MR-Bilddaten. Diese Bilddaten BD können dann beispielsweise in einem Speicher 119 hinterlegt werden.

Eine Bedienung der zentralen Steuereinrichtung 113 kann über ein Terminal mit einer Eingabeeinheit 111 und einer Anzeigeeinheit 109 erfolgen, über das somit auch die gesamte MR-Anlage 160 durch eine Bedienperson bedient werden kann. Auf der Anzeigeeinheit 109 können auch MR-Bilder angezeigt werden, und mittels der Eingabeeinheit 111 ggf. in Kombination mit der Anzeigeeinheit 109 können Messungen geplant und gestartet und insbesondere geeignete Steuerprotokolle mit geeigneten Messsequenzen wie oben erläutert ausgewählt und gegebenenfalls modifiziert werden.

Die erfindungsgemäße MR-Anlage 160 und insbesondere die Steuereinrichtung 113 können darüber hinaus noch eine Vielzahl von weiteren, hier nicht im Einzelnen dargestellten, aber üblicherweise an solchen Geräten vorhandenen Komponenten aufweisen, wie beispielsweise eine Netzwerkschnittstelle, um die gesamte Anlage mit einem Netzwerk zu verbinden und Rohdaten RD und/oder Bilddaten BD bzw. Parameterkarten, aber auch weitere Daten, wie beispielsweise patientenrelevante Daten oder Steuerprotokolle, austauschen zu können.

Wie durch ein Einstrahlen von HF-Pulsen und die Erzeugung von Gradientenfeldern geeignete Rohdaten RD akquiriert und daraus MR-Bilder BD rekonstruiert werden können, ist dem Fachmann grundsätzlich bekannt und wird hier nicht näher erläutert.

Aus dem zuvor Beschriebenen wird deutlich, dass die Erfindung wirkungsvoll Möglichkeiten bereitstellt, um ein Verfahren zur Ansteuerung eines Magnetresonanzbildgebungssystems zur Erzeugung von Magnetresonanzbilddaten bezüglich der benötigten Zeitdauer zu verbessern.

Dabei ist darauf hinzuweisen, dass die Merkmale sämtlicher Ausführungsbeispiele oder in Figuren offenbarter Weiterbildungen in beliebiger Kombination verwendet werden können.

Es wird abschließend noch einmal darauf hingewiesen, dass es sich bei den zuvor beschriebenen detaillierten Verfahren und Aufbauten um Ausführungsbeispiele handelt und dass das Grundprinzip auch in weiten Bereichen vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können. Unabhängig vom grammatikalischen Geschlecht eines bestimmten Begriffes sind Personen mit männlicher, weiblicher oder anderer Geschlechteridentität mit umfasst.

## Patentansprüche

1. Verfahren zur Ansteuerung eines Magnetresonanzbildgebungssystems zur Erzeugung von Magnetresonanzbilddaten (BD) eines Untersuchungsobjekts (O), bei dem k-Raumdaten (RD) im Rahmen einer beschleunigten Echoplanar-Bildgebung (A-EPI) mit einer Unterabtastung gemäß einem Pulssequenzschema (PSS) erfasst werden, welches Pulssequenzschema (PSS) eine Mehrzahl von Repetitionen aufweist, welche jeweils
- ein erstes Abtastschema (AS1) für eine Aufnahme (NA) von k-Raumdaten (RD) zur Nyquist-Ghosting-Korrektur oder zur Erzeugung von Magnetfeldkarten,
- ein nachfolgendes zweites Abtastschema (AS2) für eine beschleunigte Echoplanar-Aufnahme (A-EPI) und
- ein für beide Aufnahmen (NA, A-EPI) gemeinsames Anregungsschema (A-RF) umfassen,
wobei das erste Abtastschema (AS1) einer echten Teilmenge (REP1, REP2, REP3, REP4) der Mehrzahl von Repetitionen dahingehend modifiziert ist, dass auf Basis der mit dem modifizierten ersten Abtastschema (AS1) erfassten k-Raumdaten (RD) eine Ergänzung von in Folge der Unterabtastung fehlenden k-Raumdaten (RD) und/oder eine Korrektur im Bildraum von aufgrund der Unterabtastung auftretenden Artefakten ermöglicht wird.

2. Verfahren nach Anspruch 1, wobei bei der Modifikation des ersten Abtastschemas (AS1) die Zeitdauer der von der Modifikation betroffenen Repetition (REP1, REP2, REP3, REP4) unverändert aufrechterhalten wird.

3. Verfahren nach Anspruch 1 oder 2, wobei das modifizierte erste Abtastschema (AS1) und die Anzahl der von der echten Teilmenge umfassten Repetitionen (REP1, REP2, REP3, REP4) so gewählt werden, dass eine vollständige Abtastung eines k-Raum-Teilbereichs, der mindestens für eine vollständige Ergänzung der fehlenden k-Raumdaten (RD) benötigt wird, erfolgt.

4. Verfahren nach einem der vorstehenden Ansprüche, wobei das modifizierte erste Abtastschema (AS1) eine Abtastung von k-Raumlinien in Ausleserichtung (kₓ) mit unterschiedlicher Phasenkodierung (k_{y}) umfasst.

5. Verfahren nach einem der vorstehenden Ansprüche, wobei das modifizierte erste Abtastschema (AS1) eine Abtastung von k-Raumlinien in Ausleserichtung (kₓ) mit zumindest teilweise unterschiedlicher Schichtkodierung (k_{z}) umfasst.

6. Verfahren nach einem der vorstehenden Ansprüche, wobei für das modifizierte erste Abtastschema (AS1) und das zweite Abtastschema (AS2) ein Abtastschema (AS) desselben Typs verwendet wird.

7. Verfahren nach einem der vorstehenden Ansprüche, wobei zu unterschiedlichen Zeitpunkten der beschleunigten Echoplanar-Bildgebung (A-EPI) eine Aktualisierung der mit dem modifizierten ersten Abtastschema (AS1) erfassten k-Raumdaten (RD) erfolgt.

8. Verfahren nach einem der vorstehenden Ansprüche, wobei das modifizierte erste Abtastschema (AS1) umfasst:
- mindestens vier aufeinanderfolgende Phasenkodiergradienten (Gy) und
- mindestens drei aufeinanderfolgende Auslesegradienten (Gx) mit wechselnder Polarität, welche sich mit den Phasenkodiergradienten (Gy) zeitlich abwechseln.

9. Verfahren nach Anspruch 8, wobei zeitlich mit den zweiten bis vierten Phasenkodiergradienten (Gy) synchronisiert Schichtselektionsgradienten (Gz) geschaltet werden.

10. Verfahren nach Anspruch 8 oder 9, wobei die Amplituden der Phasenkodiergradienten (Gy) des modifizierten ersten Abtastschemas (AS1) in Abhängigkeit von einem Unterabtastungsfaktor des zweiten Abtastschemas (AS2) gewählt werden.

11. Verfahren nach einem der vorstehenden Ansprüche, wobei für die beschleunigte Echoplanar-Bildgebung eine der folgenden spezifischen Bildgebungstechniken genutzt wird:
- GRAPPA,
- SMS/Slice-GRAPPA,
- SENSE,
- eine Kombination aus GRAPPA und SMS,
- eine Bildgebungstechnik mit dualer Polarität.

12. Steuereinrichtung (113) für ein Magnetresonanzbildgebungssystem (160), welche zur Steuerung des Magnetresonanzbildgebungssystems (160) unter Nutzung eines Verfahrens nach einem der Ansprüche 1 bis 11 ausgebildet ist.

13. Magnetresonanzbildgebungssystem (160), aufweisend eine Steuereinrichtung (113) nach Anspruch 12.

14. Computerprogrammprodukt mit einem Computerprogramm, welches direkt in eine Speichereinheit in einen Speicher einer Steuereinrichtung (113) eines Magnetresonanzbildgebungssystems (160) ladbar ist, mit Programmcodeabschnitten, um alle Schritte eines Verfahrens nach einem der Ansprüche 1 bis 11 auszuführen, wenn das Programm in der Steuereinrichtung (113) ausgeführt wird.

15. Computerlesbares Medium, auf welchem von einer Rechnereinheit ausführbare Programmabschnitte gespeichert sind, um die Schritte eines Verfahrens nach einem der Ansprüche 1 bis 11 auszuführen, wenn die Programmabschnitte von der Rechnereinheit ausgeführt werden.

## Claims

1. Method for actuating a magnetic resonance imaging system for generating magnetic resonance image data (BD) of an examination object (O), in which k-space data (RD) is acquired as part of an accelerated echoplanar imaging (A-EPI) with undersampling according to a pulse sequence diagram (PSS), which pulse sequence diagram (PSS) has a plurality of repetitions, each of which comprising
- a first sampling diagram (AS1) for a recording (NA) of **k-**space data (RD) for Nyquist ghosting correction or for generating magnetic field maps,
- a subsequent second sampling diagram (AS2) for an accelerated echoplanar recording (A-EPI) and
- an excitation diagram (A-RF) shared by both recordings (NA, A-EPI),
wherein the first sampling diagram (AS1) of a real subset (REP1, REP2, REP3, REP4) of the plurality of repetitions is modified in that, on the basis of the k-space data (RD) acquired using the modified first sampling diagram (AS1), it is made possible to expand k-space data (RD) that is missing as a consequence of the undersampling and/or to correct artifacts occurring due to the undersampling in the image space.

2. Method according to claim 1, wherein, during the modification of the first sampling diagram (AS1), the duration of the repetition (REP1, REP2, REP3, REP4) affected by the modification is maintained in an unaltered manner.

3. Method according to claim 1 or 2, wherein the modified first sampling diagram (AS1) and the number of the repetitions (REP1, REP2, REP3, REP4) comprised by the real subset are chosen such that a k-space subregion, which is required at least for a full expansion of the missing k-space data (RD), is sampled in full.

4. Method according to one of the preceding claims, wherein the modified first sampling diagram (AS1) comprises a sampling of k-space lines in the readout direction (kₓ) with different phase encoding (k_{y}).

5. Method according to one of the preceding claims, wherein the modified first sampling diagram (AS1) comprises a sampling of k-space lines in the readout direction (kₓ) with at least partially different slice encoding (k_{z}).

6. Method according to one of the preceding claims, wherein the same type of sampling diagram (AS) is used for the modified first sampling diagram (AS1) and the second sampling diagram (AS2).

7. Method according to one of the preceding claims, wherein at different points in time of the accelerated echoplanar imaging (A-EPI), the k-space data (RD) acquired using the modified first sampling diagram (AS1) is updated.

8. Method according to one of the preceding claims, wherein the modified first sampling diagram (AS1) comprises:
- at least four successive phase encoding gradients (Gy) and
- at least three successive readout gradients (Gx) with alternating polarity, which alternate with the phase encoding gradients (Gy) over time.

9. Method according to claim 8, wherein slice selection gradients (Gz) are switched in a manner synchronised with the second to fourth phase encoding gradients (Gy) over time.

10. Method according to claim 8 or 9, wherein the amplitudes of the phase encoding gradients (Gy) of the modified first sampling diagram (AS1) are chosen as a function of an undersampling factor of the second sampling diagram (AS2).

11. Method according to one of the preceding claims, wherein one of the following specific imaging techniques is used for the accelerated echoplanar imaging:
- GRAPPA,
- SMS/Slice-GRAPPA,
- SENSE,
- a combination of GRAPPA and SMS,
- a dual-polarity acceleration technique.

12. Control facility (113) for a magnetic resonance imaging system (160), which is embodied for controlling the magnetic resonance imaging system (160) by using a method according to one of claims 1 to 11.

13. Magnetic resonance imaging system (160), having a control facility (113) according to claim 12.

14. Computer program product with a computer program, which can be loaded directly into a memory unit in a memory of a control facility (113) of a magnetic resonance imaging system (160), with program code sections for carrying out all steps of a method according to one of claims 1 to 11 when the program is executed in the control facility (113).

15. Computer-readable medium, on which program sections that can be executed by a computer unit are stored, for carrying out the steps of a method according to one of claims 1 to 11 when the program sections are executed by the computer unit.

## Revendications

1. Procédé de commande d'un système d'imagerie par résonnance magnétique pour la production de données (BD) d'image par résonnance magnétique d'un objet (O) à examiner, dans lequel on saisit des données (RD) de l'espace k, dans le cadre d'une imagerie (A-EPI) écho-planaire accélérée avec un sous-échantillonnage suivant un schéma (PSS) de séquence d'impulsions, lequel schéma (PSS) de séquence d'impulsions comporte une pluralité de répétitions, qui chacune comprennent
- un premier schéma (AS1) d'échantillonnage pour un enregistrement (NA) de données (RD) de l'espace k pour la correction Nyquist-Ghosting ou pour la production de cartes de champ magnétique,
- un deuxième schéma (AS2) d'échantillonnage venant ensuite pour un enregistrement (A-EPI) écho-planaire accéléré, et
- un schéma (A-RF) d'excitation commun aux deux enregistrements (NA, A-EPI),
dans lequel le premier schéma (AS1) d'échantillonnage d'un ensemble (REP1, REP2, REP3, REP4) partiel propre de la pluralité de répétitions est modifié en ce sens que, sur la base des données (RD) de l'espace k saisies par le premier schéma (AS1) d'échantillonnage modifié, un complément de données (RD) de l'espace k, manquant en raison du sous-échantillonnage, et/ou une correction d'artefacts se produisant dans l'espace image en raison du sous-échantillonnage, est rendu possible.

2. Procédé suivant la revendication 1, dans lequel, lors de la modification du premier schéma (AS1) d'échantillonnage, on maintient inchangée la durée des répétitions (REP1, REP2, REP3, REP4) concernées par la modification.

3. Procédé suivant la revendication 1 ou 2, dans lequel on choisit le premier schéma (AS1) d'échantillonnage modifié et le nombre de répétitions (REP1, REP2, REP3, REP4) compris par l'ensemble partiel propre, de manière à obtenir un échantillonnage complet d'une partie partielle de l'espace k, qui est nécessaire au moins pour compléter complètement les données (RD) de l'espace k manquantes.

4. Procédé suivant l'une des revendications précédentes, dans lequel le premier schéma (AS1) d'échantillonnage modifié comprend un échantillonnage de lignes de l'espace k dans la direction (kₓ) de lecture avec un codage (k_{y}) de phase différent.

5. Procédé suivant l'une des revendications précédentes, dans lequel le premier schéma (AS1) d'échantillonnage modifié comprend un échantillonnage de lignes de l'espace k dans la direction (kₓ) de lecture avec un codage (k_{z}) de tranche différent au moins en partie.

6. Procédé suivant l'une des revendications précédentes, dans lequel, pour le premier schéma (AS1) d'échantillonnage et le deuxième schéma (AS2) d'échantillonnage modifiés, on utilise un schéma (AS) d'échantillonnage du même type.

7. Procédé suivant l'une des revendications précédentes, dans lequel, à des instants différents de l'imagerie (A-EPI) écho-planaire accélérée, il s'effectue une mise à jour des données (RD) de l'espace k saisies avec le premier schéma (AS1) d'échantillonnage modifié.

8. Procédé suivant l'une des revendications précédentes, dans lequel le premier schéma (AS1) d'échantillonnage modifié comprend :
- au moins quatre gradients (Gy) de codage de phase successifs, et
- au moins trois gradients (Gx) de lecture successifs à polarité alternée, qui alternent dans le temps avec les gradients (Gy) de codage de phase.

9. Procédé suivant la revendication 8, dans lequel on prévoit des gradients (Gz) de sélection de tranche synchronisés dans le temps avec les deuxième à quatrième gradients (Gy) de codage de phase.

10. Procédé suivant la revendication 8 ou 9, dans lequel on choisit les amplitudes des gradients (Gy) de codage de phase du premier schéma (AS1) d'échantillonnage modifié en fonction d'un facteur de sous-échantillonnage du deuxième schéma (AS2) d'échantillonnage.

11. Procédé suivant l'une des revendications précédentes, dans lequel on utilise, pour l'imagerie écho-planaire accélérée, l'une des techniques d'imagerie spécifiques suivantes :
- GRAPPA,
- SMS/Slice-GRAPPA,
- SENSE,
- une combinaison de GRAPPA et de SMS,
- une technique d'imagerie à polarité duale.

12. Dispositif (113) de commande d'un système (160) d'imagerie par résonnance magnétique, qui est constitué pour la commande du système (160) d'imagerie par résonnance magnétique en utilisant un procédé suivant l'une des revendications 1 à 11.

13. Système (160) d'imagerie par résonnance magnétique, comportant un dispositif (113) de commande suivant la revendication 12.

14. Produit de programme d'ordinateur comprenant un programme d'ordinateur, qui peut être chargé directement dans une unité de mémoire dans une mémoire d'un dispositif (113) de commande d'un système (160) d'imagerie par résonnance magnétique, comprenant des parties de code de programme, pour exécuter tous les stades d'un procédé suivant l'une des revendications 1 à 11, lorsque le programme est exécuté dans le dispositif (113) de commande.

15. Support, déchiffrable par ordinateur, sur lequel sont mises en mémoire des parties de programme pouvant être exécutées par une unité informatique, afin d'exécuter les stades d'un procédé suivant l'une des revendications 1 à 11, lorsque les parties de programme sont exécutées par l'unité informatique.
